Europäisches Patentamt

European Patent Office

Office européen des brevets

(11) Publication number: **0 326 579 B1**

# EUROPEAN PATENT SPECIFICATION

(45) Date of publication of patent specification: **11.01.95**

(51) Int. Cl.⁶: **A61L 27/00**, A61L 29/00, A61L 31/00

(21) Application number: **87907467.2**

(22) Date of filing: **16.10.87**

(86) International application number:
**PCT/US87/02675**

(87) International publication number:
**WO 88/02623 (21.04.88 88/09)**

(54) **IMPROVEMENT OF THE BIOCOMPATIBILITY OF SOLID SURFACES.**

(30) Priority: **17.10.86 US 920567**

(43) Date of publication of application:
**09.08.89 Bulletin 89/32**

(45) Publication of the grant of the patent:
**11.01.95 Bulletin 95/02**

(84) Designated Contracting States:
**AT BE CH DE FR GB IT LI LU NL SE**

(56) References cited:
| | |
|---|---|
| GB-A- 2 178 963 | US-A- 3 808 113 |
| US-A- 3 959 078 | US-A- 4 378 435 |
| US-A- 4 451 568 | US-A- 4 526 909 |
| US-A- 4 589 881 | |

(73) Proprietor: **BIO-METRIC SYSTEMS, INC.**
**9932 West 74th Street**
**Eden Prairie, MN 55344 (US)**

(72) Inventor: **GUIRE, Patrick, E.**
**6741 Tartan Curve**
**Eden Prairie, MN 55344 (US)**

(74) Representative: **Parr, Ronald Edward R.E. Parr**
**& Co. et al**
**Colman House**
**Station Road**
**Knowle**
**Solihull**
**West Midlands B93 0HL (GB)**

**Description**

FIELD OF INVENTION

This invention relates to the field of biochemistry and particularly to the enhancement of the biocompatibility of various surfaces.

BACKGROUND OF THE INVENTION

The implantation of such biomaterial articles as substitute blood vessels, synthetic and intraocular lenses, electrodes, catheters and the like in and onto the body is a rapidly developing area of medicine. A primary impediment to the long-term use of such biomaterial implantables as synthetic vascular grafts has been the lack of satisfactory graft surfaces. The uncoated surfaces of synthetic blood vessels made from plastics, for example, often stimulate rapid thrombogenic action. Various plasma proteins play a role in initiating platelet and fibrin deposition on plastic surfaces. These actions lead to vascular constriction to hinder blood flow, and the inflammatory reaction that follows can lead to the loss of function of the synthetic implantable.

A "biomaterial" may be defined as a material that is substantially insoluble in body fluids and that is designed and constructed to be placed in or onto the body or to contact fluid of the body. Vascular grafts and contact lenses are examples of biomaterials.

Ideally, a biomaterial will have the following characteristics:

1. It will not induce undesirable reactions in the body such as blood clotting, tissue death, tumor formation, allergic reaction, foreign body reaction (rejection) or inflammatory reaction.

2. It will have the physical properties such as strength, elasticity, permeability and flexibility required to function as intended.

3. It can be purified, fabricated and sterilized easily.

4. It will substantially maintain its physical properties and function during the time that it remains implanted in or in contact with the body, whether it be an hour or a lifetime.

As used herein, the solid surface of a biomaterial is characterized as "biocompatible" if it is capable of functioning or existing in contact with biological fluid and/or tissue of a living organism with a net beneficial effect on the living organism. Long term biocompatibility is desired for the purpose of reducing disturbance of the host organism.

A number of approaches have been suggested to improve the biocompatibility of implantable items. One approach has been to modify the surface of a biomaterial to prevent undesirable protein adhesion by providing the biomaterial with a low polarity surface, a negatively charged surface or a surface coated with biological materials such as enzymes, endothelial cells and proteins. Solid surfaces have been coated with biochemical materials such as heparin, albumin and streptokinase to enhance thromboresistance. Albumin in particular has been physically adsorbed onto and electrostatically and covalently bound to polymer surfaces.

Munro, et. al, U.S. Patent No. 4,530,974 discloses a method of adsorbing albumin to a water-insoluble polymer such as polyurethane by covalently binding to the surface a nonionic hydrophobic aliphatic chain to which albumin will selectively bind.

Nimni et al, U.S. Patent 4,378,224 teaches a method of coating animal tissues, used to make prosthetic devices, through the formation of a three dimensional cross-linked matrix primarily composed of a calcification inhibitor.

An example of an adverse reaction that is caused by the presence of a biomaterial is the deposition of protein on contact lenses. Often contact lens wearers develop an intolerance to their contact lenses with time and this intolerance may be linked to irritation and allergic responses to biochemicals (proteins, lipids, mucopolysaccharides, and others) which deposit onto the lenses while they are worn. Current cleansing and disinfection procedures remove some of these deposits, but these procedures often leave holes and crevices in the lenses which add to the eye irritation of the wearer and serve as foci for further biochemical deposition.

Guire, U.S. Patent No. 3,959,078, describes the use of reagents to covalently bind an enzyme to aminoethyl cellulose or alkylamine glass. See, also: Guire, Stepwise Thermophotochemical Cross-linking for Enzyme Stabilization and Immobilization; Enzyme Engineering 3:63-70 (1978) and Guire, Photochemical Immobilization of Enzymes and Other Biochemicals, Methods in Enzymology XLIV:280-288 (1976). These references describe a process of covalently binding an enzyme to substrates such as chemical derivatives of controlled-pore glass, cellulose, agarose and polyacrylamides by thermochemically coupling a linking

EP 0 326 579 B1

reagent to the solid surface and photochemically coupling the enzyme to the linking reagent to provide a surface useful in the performance of in vitro diagnostic assays.

US-A-4 451 568 (Schneider) discloses an adhesive-based composition of monomers that undergo photo-polymerization to form a polymer layer that is able to adhere to a substrate. US-A-4 715 858 (Lindstrom) discloses a synthetic epicorneal lens for use in the treatment of refractive errors. The lens is of a plastic that is compatible with the cornea, may be gas permeable or metabolite permeable, and may be coated with a basement membrane material such as fibronectin or laminin.

In a first aspect the present invention provides a method of producing a biocompatible device from a biomaterial substrate having a solid surface, which comprises:

providing the solid surface with a biocompatible surface comprising a biocompatible agent by use of a chemical-linking moiety possessing a photochemically reactive group and possessing a different reactive group;

characterised in that:

in the chemical - linking moiety one of said groups is unresponsive to activation by a stimulus to which the other group is responsive;

applying stimulus to sequentially activate said groups to covalently bond said different reactive group to the molecules of the biocompatible agent and to photochemically covalently bond the linking moiety to the solid surface with a sufficient population density to enable the molecules of the biocompatible agent to effectively shield the solid surface with the biocompatible surface;

wherein the chemical linking moiety has the formula A-X-B in which:

A represents a photochemically reactive group capable, in response to specific activation, of bonding covalently to the solid surface;

B represents a different reactive group capable in response to specific activation to which group A is unresponsive, of forming a covalent bond to the biocompatible agent; and

X represents a relatively inert, non-interfering skeletal moiety joining groups "A", and "B", the skeletal moiety being resistant to cleavage in aqueous physiological fluids.

In a preferred form of the invention the biocompatible "effective" surface is formed of a plurality of separate molecules of the biocompatible agent covalently linked, through the linking moiety, to the solid surface of the biomaterial substrate to provide that surface with substantially the same biocompatible characteristics as are possessed by the biocompatible agent. The effective surface formed by the molecules of the biocompatible agent need not cover the entire surface of the biomaterial substrate: it may cover the surface in spots. For example, spots along the surface of vascular grafts may be covered by a cell attachment factor such as fibronectin. The biocompatible effective surface formed at those spots then acts as a focus for attachment of cells to the modified surface.

The "different reactive group" of the linking moiety desirably can be a thermochemical group or a photochemical group.

In a second aspect the invention provides a biocompatible device which comprises:

a substrate of a biomaterial having a solid surface and carrying molecules of a biocompatible agent;

a chemical linking moiety residue binding individual molecules of the biocompatible agent to the solid surface;

characterised in that

the chemical linking moiety residue includes a residue of a photochemically reactive group covalently bonded to the solid surface, and includes a residue of a different reactive group covalently bonded to molecules of the biocompatible agent, the photochemically reactive group residue being bonded to the solid surface so that the individual molecules of the biocompatible agent are positioned sufficiently proximate to one another as to cause said molecules to effectively shield the solid surface and to provide a biocompatible effective surface, wherein the chemical linking moiety has the formula A-X-B in which:

A represents a photochemically reactive group capable, in response to specific activation, of bonding covalently to the solid surface;

B represents a different reactive group capable, in response to specific activation to which group A is unresponsive, of forming a covalent bond to the biocompatible agent; and

X represents a relatively inert, non-interfering skeletal moiety joining groups "A" and "B", the skeletal moiety being resistant to cleavage in aqueous physiological fluids. The physiological fluid referred to is such fluid with which X will come in contact.

The biocompatible agent is chosen to enhance the function of a particular device. For example, the function of contact lenses may be enhanced by attaching molecules of polyethylene glycol to the lens surfaces to diminish the deposition of proteins on these surfaces. As another example, a cell attachment factor such as fibronectin or laminin may be bonded to a device having a polyvinyl chloride surface to

3

increase cell attachment to the device. This is desirable in the case of implantables such as catheters and substitute blood vessels.

A preferred form of the invention uses molecules of a biocompatible agent joined to one another to form a biocompatible film, and a chemical linking moiety capable of linking the film to the solid surface of the biomaterial. The chemical linking moiety includes a photochemically reactive group capable upon activation of covalently bonding to the solid surface, and the residue of a reactive group covalently bonded to the film (e.g., to the residue of the biocompatible agent molecules making up the film). One of the reactive groups is unresponsive to a stimulus to which the other reactive group responds. The method comprises activating the photochemically reactive group with a stimulus to covalently bind the biocompatible molecules. By "joined" in this context, reference is made not only to covalent bonding of adjacent biocompatible agent molecules but also to interactions caused by such forces as hydrogen bonding, ionic bonding, bonding through Van der Waals forces, and the like.

The biocompatible agent having molecules joined to one another to form a film may comprise molecules of one agent or it may comprise molecules of two or more agents such as heparin and albumin.

## DESCRIPTION OF THE PREFERRED EMBODIMENTS

The solid surface that is rendered biocompatible in accordance with the invention desirably is of a synthetic or natural material that is insoluble in physiological fluids. The surface may be one or more surfaces of devices intended to function in contact with tissue and/or fluids of living organisms. The solid surface of the device may be any suitable metal such as polished titanium or stainless steel or a polymer such as polyurethane, polyvinylpyrrolidone, silicone elastomers, polyethylene, polytetrafluoroethylene, poly-(p-phenyleneterephthalamide), polyvinyl chloride, polypropylene, polyolefins, polyesters, polyacrylates (including polymethacrylates); minerals or ceramics such as hydroxyapatite; human tissue such as bone, skin and teeth; organic materials such as wood, cellulose and compressed carbon; and other natural and synthetic materials such as glass, rubber, wood and the like. Examples of devices which may be provided with biocompatible surfaces in accordance with this invention include vascular graft tubing, dialysis tubing or membrane, blood oxygenator tubing or membrane, ultrafiltration membrane, intra-aortic balloon, blood bag, catheter, suture, soft or hard tissue prosthesis, synthetic prosthesis, artificial organs, and lenses for the eye such as contact and intraocular lenses.

The solid surface is desirably thermochemically unreactive. "Thermochemically unreactive" means that the surface is free of any surface treatment designed to increase the ability of the surface to thermochemically react. Examples of thermochemically unreactive surfaces include polytetrafluroethylene, polyethylene, polypropylene, polyvinyl chloride, polyvinylpyrrolidone silicone elastomers, stainless steel and titanium.

Molecules of a biocompatible agent are attached to the surfaces of the biomaterial substrates to improve biocompatibility. The biocompatible agent may be a growth factor such as endothelial cell growth factor, epithelial cell growth factor, osteoblast growth factor, fibroblast growth factor, platelet derived growth factor, neural growth factor, or angiogenin growth factor; an antimicrobial agent such as lysosyme or penicillin; an antithrombogenic agent such as heparin, albumin, streptokinase, tissue plasminogin activator (TPA) or urokinase; a thrombogenic agent such as collagen or a hydrophilic polymer such as polyethylene glycol, hyluronic acid, chitosan or methyl cellulose, and other proteins, carbohydrates and fatty acids. The biocompatible agent may comprise molecules of one of the above listed agents or it may comprise molecules of two or more agents. For example, the biocompatible agent may comprise molecules of both albumin and heparin.

In one embodiment the molecules of a biocompatible agent are joined to one another to form a film that is attached to a solid surface by a linking moiety. The biocompatible agent desirably may be hyaluronic acid or albumin. A biocompatible device having a film attached may be an artificial hip joint coated with a film of hyaluronic acid.

With reference to the chemical linking moiety A-X-B, X is preferably: a $C_1$-$C_{10}$ alkyl group such as polymethylene; a carbohydrate such as polymethylol; a polyoxyethylene such as polyethylene glycol; or a polypeptide such as polylysine.

The reactive group B is a group that upon suitable activation covalently bonds to proteinaceous or other biocompatible agents. Such groups are typified by thermochemical groups and photochemical groups, as described and exemplified in Guire, U.S. Patent 3,959,078.

The photochemically reactive groups (A) (the covalent bonding of which is activated by actinic radiation) may be typified by aryl, alkyl and acyl azides, oxazidines, isocyanates (nitrene generators), alkyl and 2-ketodiazo derivatives and diazirines (carbene generators), aromatic ketones (triplet oxygen generators),

4

aromatic diazonium derivatives and numerous classes of carbonium ion and radical generators. Reference is made to Frederick J. Darfler and Andrew M. Tometsko, chapter 2 of Chemistry and Biochemistry of Amino Acids, Peptides and Proteins (Boris Weinstein, ed) vol. 5, Marcel Dekker, Inc. New York, 1978, for further description of photochemically reactive groups. Azidonitrophenyls, fluoroazido nitrobenzenes, and aromatic ketones form a preferred group due to their stability to chemical reaction conditions in the dark and their susceptibility to activation by light of wave lengths harmless to most biomaterials, to form short-lived reactive intermediates capable of forming covalent bonds in useful yield with most sites on the biomaterial.

Nitrophenylazide derivatives (shown as including the - X - group) appropriate for use as photochemically reactive groups for the most part can be derived from fluoro-2-nitro-4-azidobenzene, and include 4-azido-2-nitrophenyl(ANP)-4-amino-butyryl, ANP-6-aminocaproyl, ANP-11-aminoundecanoyl, ANP-glycyl, ANP-aminopropyl, ANP-mercaptoethylamino, ANP-diaminohexyl, ANP-diaminopropyl, and ANP-polyethylene glycol. ANP-6-aminocaproyl, ANP-11-aminoundecanoyl, and ANP-polyethylene glycol are preferred. Aromatic ketones preferred for use as photochemically reactive groups include benzylbenzoyl and nitrobenzylbenzoyl.

Thermochemical reactive groups (that are activated by heat energy) are typified by and include nitrophenylhalides, alkylamino, alkylcarboxyl, alkylthiol, alkylaldehyde, alkylmethylimidate, alkylisocyanate, alkylisothiocyanate and alkylhalide groups.

Groups appropriate for use as thermochemically reactive groups include carboxyl groups, hydroxyl groups, primary amino groups, thiol groups, maleimides and halide groups. N-oxysuccinimide carboxylic esters of such groups as 6-amino hexanoic acid and amino undecanoic acid, alkylthiol groups such as mercaptosuccinic anhydride and beta-mercaptopropionic acid, homocysteinethiolactones, and polyetheylene glycol derivatives are preferred.

The devices of this invention have biocompatible solid surfaces that include molecules of a biocompatible agent and a chemical linking moiety residue. The chemical linking moiety residue possesses a residue of a photochemically reactive group bonded to the solid surface and possesses a residue of a different reactive group covalently bonded to the biocompatible agent. The residue of the photochemically reactive group is that portion of a photoreactive group (described above) represented by "A" in the general formula, that remains after a covalent bond has formed. When the photoreactive group is ANP and the solid substrate is polyethylene, the residue is a carbon-nitrogen bond. A nitrene formed when ANP is light-activated, reacts with a carbon of the polyethylene to form a covalent bond. When the photoreactive group is BBA and the solid substrate is polyethylene, the residue is a carbon-carbon bond. When BBA is stimulated by light, the carbon joining the two phenyl groups is activated to a triplet state causing a carbon-carbon bond to form between the polyetheylene and the BBA and to form an hydroxyl group. When the different reactive group ("B") is NOS the residue is the carboxyl carbon of that group bonded to an oxygen or nitrogen group of a biocompatible agent such as fibronectin.

As enzymes, cell attachment factors and certain other substances that may be employed as biocompatible agents are somewhat sensitive to high temperatures, it is desired that the different reactive group on the linking moiety employed in the present invention be activated by (that is, undergo covalent bonding in response to) easily applied and nonharmful stimuli such as moderate heat (e.g., body temperature or below) and light. Reactive groups that are unresponsive to the stimulus to which the photochemically reactive group responds may be groups that react to changes in pH, or to the addition of another chemical species and the like.

As stated above, the chemical linking moiety covalently bonds to said solid surface in such population density to enable the molecules of the biocompatible agent effectively to shield the solid surface with the biocompatible surface. The density of bound chemical moieties necessary to provide an effective surface varies with the particular biocompatible agent used.

The invention may be better understood by reference to the following non-limiting examples. Table 1 is a list of abbreviations of terms used in the following descriptions.

## TABLE 1:   LIST OF ABBREVIATIONS

| Abbreviation | Full Name |
|---|---|
| EGF | Endothelial growth factor |
| PEG | Polyethylene glycol |
| PVC | Polyvinyl chloride |
| PE | Polyethylene |
| PP | Polypropylene |
| PTFE | Polytetrafluoroethylene |
| FNAB | Fluoronitroazidobenzene |
| ANP | 4-azido-2-nitrophenyl |
| KOH | Potassium hydroxide |
| TLC | Thin layer chromatography |
| NOS | N-oxysuccinimide |
| EAC(A) | Epsilon amino caproic acid |
| AUD(A) | Amino undecanoic acid |
| BBA | Benzoyl benzoic acid |
| DMSO | Dimethyl sulfoxide |
| DMF | Dimethylformamide |
| EDC | 1-ethyl-3-(3-dimethylamino-propyl) carbodimide |
| PEG-1000 | Polyethylene glycol, molecular weight 1000 |
| PEG-4000 | Polyethylene glycol molecular weight 4000 |
| PBS | Phosphate buffered saline |
| FN | Fibronectin |
| COL | Collagen |
| nBBA | Nitrobenzoylbenzoic acid |
| HEPES | N-2-hydroxyethylpiperazine - N'-2-ethane sulfonic acid |
| HSA | Human serum albumin |
| HGG | Human gamma globulin |
| LYZ | Lyzosyme |
| mmole | millimole |

## TABLE 1 Continued: LIST OF ABBREVIATIONS

| Abbreviation | Full Name |
|---|---|
| ml | milliliter |
| $MW_{app}$ | Approximate molecular weight |
| mg | milligram |
| M | Molar |
| pmole | picomole |
| ng | nanogram |
| ug | microgram |
| ID | Inner diameter |

Example 1

Endothelial Cell Attachment/Growth

1. Plastic Surfaces. Various cell factors were coupled to polymeric surfaces tested in vitro to determine the effect of these factors upon cell attachment and overgrowth. The polymeric surfaces included polyvinyl chloride (PVC), polyethylene (PE), polypropylene (PP) and polytetrafluoroethylene (PTFE) GORE-TEX (6mm reinforced expanded PTFE, a trademarked product of W. L. Gore and Associates, Inc.). Commercial tubing tested included polyester (Dacron, D, 6mm straight knitted dacron velour, a trademarked product of Dupont), silicone elastomer, (Silastic $^R$, S, 0.03 I.D., tubing, a trademarked product of Dow Corning) and polyurethane. Polystyrene plates were used as controls.

2. Preparation of the Chemical Linking Moiety. The chemical linking moiety used in these examples were the N-oxysuccinimide (NOS) esters of 4-azido-2-nitrophenyl epsilon amino caproic acid (ANP-EACA), 4-azido-2-nitro-phenyl amino undecanoic acid (ANP-AUDA) and benzoylbenzoic acid (BBA). ANP-EAC-NOS and ANP-AUD-NOS were prepared by the method described in P. Guire, D. Fliger and J. Hodgson, "Photochemical Coupling of Enzymes to Mammalian Cells", Pharmacological Research Communications, Vol. 9, pp 131-141 (1977), incorporated herein by reference. Briefly, fluoro-2-nitro-4-azido benzene was reacted with either epsilon amino caproic acid or amino undecanoic acid to substitute the relatively poorly reactive fluoride with an amino alkyl carboxy group. The carboxy group was then esterified, by carbodiimide activation with N-hydroxy succinimide to yield the N-oxysuccinimide carboxylic ester. The NOS ester of benzoylbenzoic acid was prepared by esterifying the carboxy group by carbodiimide activation with N-hydroxy succinimide.

When ANP-EAC-NOS or ANP-AUD-NOS is the chemical linking moiety used, the ANP group is the photochemically reactive group represented by the letter "A" in the A-X-B general formula discussed above. The NOS group is the different reactive group represented by the letter "B" in the formula. The EAC or AUD group acts as the spacer between the two reactive groups and is represented by the X in the general formula. When BBA-NOS is the chemical linking moiety, the benzoylbenzoic group is the photochemically reactive group represented by the "A" in the general formula and the NOS group is the different reactive group represented by the letter "B". The group represented by X is the carbon connecting the two reactive groups.

3. Covalent Bonding of Growth Factors to the Chemical Linking Moieties. The biocompatible agents fibronectin, laminin, collagen, endothelial growth factor, and human serum albumin were tested for their abilities to promote endothelial cell attachment and overgrowth on synthetic biomaterials. These agents were coupled to the N-oxysuccinimide (NOS) esters of 4-azido-2-nitrophenyl epsilon amino caproic acid (ANP-EACA), 4-azido-2-nitrophenyl-undecanoic amino acid (ANP-AUDA) or benzoylbenzoic acid (BBA) as follows. As used herein "photolabeled" refers to a biocompatible agent that has been coupled to a chemical linking moiety by the different reactive group and that has a photochemically reactive group.

A. Covalent Binding of Fibronectin and Laminin to the Linking Moiety. Human fibronectin (University of Wisconsin Medical School) and mouse laminin (obtained from Bethesda Research Lab.) were separately dissolved at 1 mg/ml concentrations in 0.1M borate, pH 9.0. A solution of ANP-EAC-NOS in dry dimethylformamide ("DMF"), ANP-AUD-NOS in dry DMF, or BBA-NOS in dry dioxane was slowly added to the fibronectin or laminin solution in equamolar amounts to the concentration of protein epsilon amino groups (lysine residues) by syringe drive at 4°C in the dark over 16 hours. Then the mixture was stirred 4 hours in the cold. The fibronectin or laminin solution was centrifuged to remove insoluble material then applied to a Sephadex G-75 column to remove uncoupled photoreagent. The fractions were monitored at 260nm and 462nm to assess the photogroup/protein ratios.

B. Covalent Binding of EGF, Collagen, and HSA to Chemical Linking Moieties. Human placenta Type IV collagen (Sigma Pharmaceutical), endothelial growth factor (Sigma Pharmaceutical), and human serum albumin (Sigma Pharmaceutical) were separately dissolved at 2 mg/ml concentrations in 0.1M borate, pH 9.0. solution of ANP-EAC-NOS in DMF, ANP-AUD-NOS in DMF or BBA-NOS in dioxane was slowly added to the solution containing the biocompatible agent in 5X molar amounts to the concentration of epsilon amino groups (lysine) residues by syringe drive at 4°C in the dark over 16 hours. Then the solution was dialyzed against 4 1000ml changes of phosphate buffered saline (PBS) and centrifuged to remove insoluble material. The product was analyzed at 260nm, 280nm and 462nm to assess the photogroup/protein ratios.

4. Covalently Binding the Biocompatible Agents to Plastic Surfaces. Various sheets, tubes and flat pieces of polyethylene, polyvinyl chloride, polypropylene, polyurethane, Dacron[R] (velour), Silastic[R] (medical grade), and polytetrafluroethylene above were used. A 0.05ml aliquot of solutions containing 0 to 500 $\mu$g/ml of photolabeled biocompatible agent was added to each 0.5 cm$^2$ section of plastic surface. The solution was allowed to adsorb onto each piece for 3 hours at room temperature in the dark. The excess liquid was removed and the biocompatible agents were covalently linked to the surfaces by photolysis for 12 hours at the appropriate wavelength (Tungsten "spotlite" for ANP and long wavelength UV for BBA). After photolysis, the specimens were washed with a 4 second stream of PBS to remove non-covalently linked molecules of photolabeled biocompatible agent. The pieces were then placed in tissue culture to assess the endothelial cell reaction to the cell factors as follows.

5. In Vitro Tests Performed with Modified Surfaces.

A. Radio-labeled biocompatible agents. Radiolabeled [³H] biocompatible agents were photolabeled as described above and photocoupled to plastic surfaces. The plastics surfaces were extensively washed with PBS, then dissolved in organic solvent, and counted by liquid scintillation spectrometry. Some representative results are given in the Table 2.

Table 2

| Sample results of amounts of growth factors photocoupled to various materials | | | | |
|---|---|---|---|---|
| Photolabeled Biocompatible Agent | Solid Surface | Ng Growth Factor Applied/cm$^2$ | Ng Growth Factor Photocoupled/cm$^2$ | % Coupling Efficiency |
| ANP-EAC-FN | PVC | 843.04 | 677.6 | 80.4% |
| | Polyurethane | 843.04 | 823.04 | 97.6% |
| BBA-FN | PVC | 3564.0 | 1091.2 | 30.62% |
| | Polyurethane | 3564.0 | 2622.4 | 73.58% |
| ANP-EAC-COL | PVC | 2675.2 | 374 | 14.0% |
| | Polyurethane | 2675.2 | 2173.6 | 81.26% |
| BBA-COL | PVC | 1105.2 | 197.56 | 17.9% |
| | Polyurethane | 1105.2 | 920.3 | 83.3% |

As these results show the photo labeled biocompatible agents covalently coupled to these surfaces.

B. Attachment of Bovine Endothelial Cells to Modified Plastic Surfaces. Bovine endothelial cells were collected from fetal animals 8-24" in length. Cornea, aorta and umbilical endothelial cells were harvested aseptically. Cells were grown in a 5% CO₂ incubator at 37°C in a known high glucose cell growth medium such as Dulbecco's modified Eagle's medium (described in R. Dulbecco and G. Freeman, Virology, Vol. 8:396 (1959) and J. D. Smith, G. Freeman, M. Vogt and R. Dulbecco, Virology, Vol. 12:185-196 (1960)) with 25mmole HEPES buffer, 10% bovine calf serum, and 2.5 micrograms amphotericin B/ml (the "growth media"). Once the plates, tubes or sheets were prepared; cell cultures were prepared from primary cell lines. The cells were detached from the cell culture surface with a 0.25% solution of trypsin and resuspended in the growth media. Cells were then counted using a trypan blue (0.4%) solution and a hemocytometer. Various concentrations of cells were layered on the prepared materials. Cell attachment was monitored for various time periods from 5 minutes to 14 days. Attachments were determined by at least two methods. In one, sample materials were removed from culture media and washed 2 times with sterile saline. Cell stains were then applied and total cells on the surface were counted. A second method was to trypsinize the cells off the surface with a trypsin solution and count using the trypan blue method.

Representive results of the attachment and outgrowth of endothelial cells on precoated polyvinyl chloride plastic pieces are reported in Table 3. The number of viable cells attached to each piece were determined by trypan blue staining procedures.

Table 3

| Cell attachment determinations and outgrowth of endothelial cells on the treated polyvinyl chloride surface. | | | | |
|---|---|---|---|---|
| Biocompatible agent and chemical-linking moiety | Ng growth factor/cm$^2$ | 3-day cell counts* | 7-day cell counts | 7-day outgrowth* |
| ANP-EAC-FN | 677.6 | 2610(2 + -3 + ) | 3539(2 + -3 + ) | 1.5-1.75mm(2 + ) |
| BBA-FN | 1091.2 | 868(1 + -2 + ) | 14500(2 + ) | 3.75-4.25mm(2 + -3 + ) |
| ANP-EAC-COL | 374.0 | 14500(3 + -4 + ) | 15833(2 + -3 + ) | 1.0-3.0mm(3 + ) |
| BBA-COL | 197.6 | 5749(2 + ) | 21500(3 + ) | 2.5-4.0mm(3 + ) |
| Control PVC | | 0 | 7(2 + ) | 1.5-2.0mm(3 + ) |

C. Attachment of Human Umbilical Endothelial Cells. Primary human endothelial cells were harvested from fresh (less than 4 days old) human umbilical cords. Cords were rinsed with 20 mls-cord buffer (0.144M NaCl, 0.004M KCl and 0.001M PO$_4$) twice to remove blood and clots. Collagenase was pushed into the cord and left for 20 minutes at room temperature. Using 10 ml of warm cord buffer, the collengenase and detached cells were flushed into tubes. The suspension in the tubes were combined and centrifuged at 1500 rpm for 5-10 minutes. The supernatant was poured off and the cells resuspended in 10 ml of cord buffer. Following the second centrifugation, the cells were resuspended in cord buffer and plated into tissue culture disks. All cells were incubated in 37°C incubator with 5% CO$_2$. Cells were radiolabeled using $^{51}$Cr in cord media without calf serum.

Labeled cells were then used for cell attachment studies. Plates, sheets, and tubes of the plastics described above were prepared as recorded above. The cells were trypsinized and counted with the trypan blue method. Cells were allowed to adhere to the prepared plastic for three hours to seven days. Cells were rinsed off and the total number of any remaining attached cells were compared to the number of non-attached cells. Representative results appear in Table 3 above.

D. Outgrowth Measurement using Endothelial Cells. The following techniques were used to monitor the outgrowth of cells from a point of origin as the cells grow to cover the surfaces of the plastics listed above modified as follows. Solutions of biocompatible agents containing from 0 to 500 micrograms of cell factors were coated onto surfaces from 1 to 6 cm long to establish a gradient. Cells were not detached from the tissue with trypsin or any proteinase. The tissue was placed on a point of origin at the low end of the gradient and marked. The tissue was allowed to sit for 15 minutes at room temperature. Growth media was added to give a moist coating over the plastic. All protocols were carried out using aseptic conditions. Plates were then incubated at 37°C in a 5% CO$_2$ incubator. Outgrowth was measured daily for up to two weeks or until the length of plastic was completely covered. Outgrowth on the treated surfaces was compared to nontreated control surfaces as reported in Table 3 above. All materials were rinsed and stained for scanning electron microscopy.

These results demonstrated that the covalent attachment of the growth factors fibronectin (FN) and collagen (COL) to the plastic surface improved the biocompatibility of the plastic with bovine endothelial cells. The cells preferentially attached to the modified surfaces versus control surfaces as is indicated by the distance they grew out over the plastic surface.

6. In Vivo Studies.

Two conditioned dogs were obtained for this study. Pieces of GORE-TEX (reinforced expanded polytetrafluroethylene, a trademarked product of W. L. Gore and Associates, Inc.) were precoated with FN, ANP-EAC-FN (adsorbed and photocoupled), ANP-EAC-FN (adsorbed only) and a PBS-control. The testing was a blind study. The grafts were labeled by lettering; however, the surgical team did not know which grafts had modified surfaces and which were controls. Each dog received two 6mm x 5cm pieces of GORE-TEX implanted in the left and right iliac artery. The grafts were left in the dogs for one month (30 days). The dogs were given the anti-inflammatory drugs Persantine and Aspirin to mimic human implant procedures.

Both control grafts (PBS and FN) were patent and had sufficient caliber. The anastomatic lines were intact and the inner surfaces were smooth. Endothelialization was incomplete with the control grafts. There appeared to be no evidence of significant thrombosis. Both grafts to which ANP-EAC-FN was bound (photocoupled and adsorbed only) were free of thrombosis formation. Endothelialization was complete giving the inner surfaces of the grafts a smooth, shiny appearance.

Example 2

Modification of the Surfaces of Contact Lenses and Introcular Lens Implants

The experiments described in this example involved preparations of hydrophilic polymers (a biocompatible agent) bound to a chemical linking moiety, photocoupling the moieties to contact lens surfaces, and measuring the in vitro protein deposition from artificial tear solutions onto these lenses in comparison to non-treated lenses. Experiments to study the compatibility of the biocompatible agents in vitro with corneal pieces and in vivo in rabbit eyes were conducted to assure that there were not toxic or irritant reactions to the resulting lenses.

1. Binding Biocompatible Agents to the Chemical Linking Moiety.

A. Preparation of Photolabeled Polyethylene Glycols. Polyethylene glycols of molecular weights 1000 (PEG-1000) and 4000 (PEG-4000) were labeled with fluoronitroazidobenzene (FNAB) by modification of the phase transfer method of "Kimura, and S. Regen, Journal of Organic Chemistry 48; 195 (1983) the teachings of which are incorporated by reference herein. Briefly, the phase-transfer synthesis of 4-azido-2-nitrophenyl polyethylene glycol (ANP-PEG) involved the mixture of 60%- aqueous potassium hydroxide ("KOH")/toluene with FNAB and PEG, followed by extraction and thin layer chromatographic (TLC) purification as described below.

ANP-PEG-1000. ANP-PEG-1000 was prepared by adding 0.05 mmole PEG-1000 to 5 mls 60% KOH and 0.5 mmole FNAB to 10 ml toluene. This reaction mixture was rapidly stirred at room temperature for 16 hours. The product was isolated from the organic layer. TLC in 85/15/1/1 chloroform/methanol/$H_2O$/acetic acid or ammonium hydroxide separated mono-and di-substituted derivatives of ANP-PEG-1000 from unlabeled PEG. The band corresponding to ANP-PEG-1000 (lower $R_f$ value) was extracted from silica gel with TLC solvent and azeotrophed to remove residual acid or base. The final product was soluble in water and resulted in the conversion of 30-40% of the PEG starting material to ANP-PEG-OH product.

ANP-PEG-4000. The ANP-PEG-4000 was prepared by the same procedure as that described above except that the reaction mixture was rapidly stirred at 50°C to ensure all reagents remained in solution during the course of the reaction. The yield of ANP-PEG-4000-OH was 10%.

B. Preparation of Photolabeled Jeffamines. Polyoxypropylenepolyamines and polyoxyethylenepolyamines (referred to as "Jeffamines", a trademark of Jefferson Chemical Co., Inc.) were photolabeled by coupling the N-oxysuccinimide ("NOS") esters of ANP-EACA, BBA and nBBA to the polymers. These NOS-derivatives were added in 0.5X amounts to 1X Jeffamine in very dry (high purity) solvents (ANP-EAC-NOS in dry tetrahydrofuran, BBA-NOS in dry dioxane or dimethylformamide and nitro BBA-NOS in dry dioxane or dimethylformamide). After 16 hours of reaction at room temperature in the dark, the products were isolated by TLC in 85/15/1/1/ chloroform/methanol/$H_2O$/acetic acid. Monosubstituted Jeffamine derivatives were extracted with the TLC solvent and azeotrophed with water to remove the residual acetic acid. The water-soluble products ANP-EAC-Jeffamine, BBA-Jeffamine, and nBBA-Jeffamine were isolated in 15%, 10% and 12% yields, respectively.

C. Preparation of ANP-Hyaluronic Acid. The terminal sugar of human placental hyaluronic acid ($MW_{app}$100-130,000) was activated by the periodate procedure described in E. Junowicz and S.E. Charm, "The Derivatization of Oxidized Polysaccarides for Protein Immobilization and Affinity Chromatography," Biochimica et Biophysica Acta, Vol. 428: 157-165 (1976) and incorporated herein by reference. This procedure entailed adding sodium or potassium periodate to a solution of hyaluranic acid thus activating the terminal sugar. The hyaluronic acid was added to a 10-fold excess of Jeffamine and allowed to react 4 hours at room temperature. The linkages were stabilized by reduction with sodium cyanoborohydride, followed by exhaustive dialysis to remove most of the excess Jeffamine. A 10-fold molar excess of ANP-EAC-NOS in DMF was added to the Jeffamine-hyaluronate in 0.1 M carbonate, pH 9.0, by syringe drive. This addition required 16 hours and was conducted at room temperature in the dark. The excess ANP-EAC-NOS and ANP-EAC-Jeffamine was removed by gel filtration chromatography. The integrity of the azide group, which is required for photocoupling of the moiety to the contact lens polymer backbone, was analyzed by infrared spectroscopy to detect the ANP group, a polyethylene glycol assay to detect the Jeffamine spacer, and a modified carbazole assay described in T. Bitter and H. Muir, Analytical Biochemistry Vol. 4: 330-334 (1962) and incorporated herein by reference to determine the uronic acid content of the derivative.

The polyethylene glycol assay was developed using the Dragendorff reagent (tetraiodobismuthic acid-barium chloride). A 5-ml portion of stock reagent (425-mg bismuth nitrate, 10-gm potassium iodide in acetic acid and water) was added to 10-ml 10% barium chloride in water and a background reading at 516+nm was noted. Then 0.1-ml of the sample was added and the contents mixed by inversion of the cuvette. A reading was taken at 516-nm after 1 minute of incubation. The values were compared to a standard curve.

The carbazole assay was performed as follows. A 3.0 ml portion of sulfuric acid reagent (0.025M sodium tetraborate-10 $H_2O$ in sulfuric acid) was cooled to -70°C. A 0.5 ml portion of sample was layered onto the acid and the mixture was stirred (30 min.) until it reached room temperature. The tubes were heated at 100°C. (10 min.), a 0.1 ml aliquot of carbazole reagent (0.125% carbazole in absolute ethanol) was added, the tube contents were mixed (5 min.), heated at 100°C. (15 min.), then cooled to room temperature in an ice bath. The samples were analyzed spectrophotometrically at 530 nm against a sulfuric acid reagent blank. The results were compared to a standard curve constructed with 4-40 ug/ml glucuronolactone standards. The assay was sensitive to detecting 20 pmole of hyaluronic acid.

The fractions containing one ANP, one Jeffamine and one hyaluronate molecule were pooled and used as a biocompatible agent.

D. Preparation of Photolabeled Hyaluronic Acid, Methyl Cellulose and Chondroitin Sulfate. ANP-EAC-Jeffamine, BBA-Jeffamine and nitro-BBA-Jeffamine were linked to the carboxyl groups of uronic acid residues of hyaluronic acid and chondroitin sulfate by a carbodiimide procedure as follows. A 5 molar excess of photolabeled Jeffamine and 1-ethyl-3-(3-dimethylaminopropyl) carbodiimide with HCl was mixed with the polysaccharide polymer in water adjusted to pH 4.5 with 0.1N HCl. The mixture was allowed to react at room temperature in the dark for 24 hours. The product was purified by gel filtration chromatography then analyzed for photogroup and carbohydrate content as described above.

E. Preparation of Photolabeled Collagen. Human placenta Type IV collagen (available from Sigma Pharmacueticals) was dissolved at a 1 mg/ml concentration in 0.1M borate, pH 9.0. ANP-EAC-NOS in DMF, BBA-sulfo-NOS in dioxane or nitro BBA-NOS in dioxane was slowly added to the collagen solution in a 50x molar excess by syringe drive at 4°C in the dark over 16 hours. After the addition was complete, the mixture was stirred 4 hours in the cold. The collagen product was dialyzed against 4 changes of PBS then centrifuged to remove insoluble material. The supernatant was measured spectrophotometrically at 260 nm, 280 nm and 462 nm to assess the photogroup/protein ratio.

F. Preparation of Photolabeled Proteinases. ANP-EAC-NOS, BBA-NOS and nBBA-NOS photogroup dissolved in organic solvent at 25 mg/ml concentrations, were added in 50 molar excess to papain (papaya, MW 23,426) by syringe drive at 4°C in the dark over 16 hours. After addition of the photogroup was completed, the mixture was stirred an additional 4 hours, then dialyzed in PBS to remove uncoupled photogroups. After dialysis, the product was centrifuged to remove insoluble material. The supernatant was measured spectrophotometrically at 260 nm, 280 nm, and 462 nm to estimate the photogroup/protein ratio.

2. Photocoupling Biocompatible Agents to Lens Surfaces. The photolabeled biocompatibles agents obtained above were added to the contact lens materials described in Table 4 at a concentration of 250-1000 pmole agent/contact lens. The solution was allowed to adsorb onto the contact lenses at room temperature in the dark for 3 hours. The photolabeled agents were then covalently linked to the plastic by photolysis for 12 hours at the appropriate wave length (450 nm for ANP and 320 nm for BBA and nBBA derivatives). After photolysis the contact lenses were washed with 5x5 ml of normal saline (0.85% NaCl) to remove non-covalently linked groups.

Radiolabeled groups may be coupled to the lens materials, and the lens pieces treated with tetrahydrofuran followed by DMSO to release the radiolabel from the solid surface. Scintillation fluor is then added and the amount of biocompatible agent/cm$^2$ determined by liquid scintillation spectroscopy. Representative results are shown in Table 4.

Table 4

| Load Densities of Biocompatible Agents on Various Contact Lens Materials | | | | |
|---|---|---|---|---|
| Biocompatible Agent | Contact Lens Material | *pmole/cm$^2$ | ng/cm$^2$ | Coupling Efficiency |
| ANP-1000 | Polyvinyl chloride | 19.96 | 19.96 | 22.86% |
| | Sofspin (polymacon) | 33.45 | 33.45 | 12.95% |
| | Permaflex | 33.97 | 33.97 | 12.90% |
| | Vistamarc | 34.26 | 34.26 | 13.20% |
| | Lidofilcon | 63.12 | 63.12 | 24.30% |
| | Silicone | 33.97 | 33.97 | 4.80% |
| | **Polymacon Button | 2408.60 | 2408.60 | 38.23% |
| ANP-4000 | Sofspin (polymacon) | 27.06 | 108.24 | 10.50% |
| | Permaflex | 42.86 | 171.44 | 16.50% |
| | Silicone | 170.60 | 682.40 | 22.70% |
| | **Polymacon Buttons | 1574.00 | 6296.00 | 25.00% |
| nitro BBA-2000 | Polyvinyl Chloride | 23.20 | 46.40 | 26.60% |
| | Sofspin | 13.14 | 26.28 | 5.10% |
| | Permaflex | 8.21 | 16.42 | 3.20% |
| | Silicone | 95.61 | 191.22 | 13.50% |
| | **Polymacon Buttons | 3738.00 | 7476.00 | 56.45% |
| BBA-2000 | Silicone | 113.20 | 226.40 | 15.60% |
| | **Polymacon Buttons | 4035.10 | 8070.20 | 64.30% |
| ANP-Hyaluronic acid | Silicone | 25.00 | 25.00 | 7.00% |
| | **Polymacon Buttons | 130.95 | 130.95 | 7.90% |

* Values were averaged from replicates of 10

** Polymacon loads are based on total volume, cm$^3$, rather than surface area.

Sofspin contacts are made of polymacon (polymethacrylate) with about 38.6% water and are a trademarked product of Bausch & Lomb, Inc.

Permaflex contacts are made of polymethacrylate with about 74% water and are a trademarked product of Coopervision, Inc.

Vistamarc contacts are made of polymethacrylate with about 58% water and are a trademarked product of Johnson & Johnson.

Lidofilcon contacts are made of polymethacrylate with about 70% water and are a product of Bausch & Lomb, Inc.

The values in Table 4 are expressed as pmole biocompatible agent per square centimeter surface area or ng/cm$^2$.

The coupling efficiencies were based upon addition of 260 pmole/cm$^2$ biocompatible agent/contact lens materials, 710 pmole/cm$^2$ biocompatible agent/silicone, and 660 pmole/cm$^3$ biocompatible agent/cm$^3$ polymacon button. ANP-hyaluronate was added at 357 pmole/cm$^2$ to silione and at 1655 pmole/cm$^3$ to polymacon button material. The ANP derivatives coupled at higher load densities than the nBBA-Jeff on the hydrogel contact lens materials. These results were reversed for the silicone compound.

3. In Vitro Protein Adsorption Studies. Artificial human tears were prepared according to the formula found in B.P. Gloor, "The Lacrimal Apparatus" in Adler's Physiology of the Eye: Clinical Applications - (R.A. Moses, ed.), C.V. Mosby Co., St. Louis, MO (1981) the teachings of which are incorporated herein. As indicated in that reference the major proteins present in human tears are serum albumin (HSA), gamma-globulin (HGG), and lysozyme (LYZ). The major sterols present in human tears are cholesterol and cholesterol esters.

A. ³H Proteins. The protein components were tritiated by reductive methylation with formaldehyde and tritiated sodium borohydride as described in N. Jentoft and D. C. Dearborn, Journal of Biochemistry, Vol. 254: 4359-4365 (1979) and incorporated herein by reference. Briefly, the biocompatible agent in 1mg/ml concentration in 0.1M HEPES, pH 7.4 was methylated by formaldehyde reacting with tritiated sodium borohydride and rocking at 22°C for about 2 hours. The product was dialyzed against PBS in 0.01M phosphate, 0.15M sodium chloride, pH 7.4, and affinity purified on gelatin sepharose. Bound agent was eluted with 1M sodium bromide 0.02M sodium acetate, pH 5.0 then dialyzed against PBS, pH 7.4.

B. Preparation of Artificial Tears. The radiolabeled proteins described above were used in preparation of artificial tears. One of the radiolabeled proteins or the tritiated cholesterol was included in each tear mixture. The other components were not radiolabeled. The contact lens materials were incubated in the artificial tear solution for one week at 37°C with gentle agitation. At the end of this time the lens materials were washed with 5 x 10ml of 0.85% NaCl. The amount of protein adsorbed to the lens materials was then determined by liquid scintillation counting.

In vitro protein deposition results are given in Table 5. Reduction in total protein deposition reached 85% in ANP-1000-OH modified Sofspin lenses. Individual biocompatible agents increased or stayed at the same levels as some of the control contact lens materials, but the overall protein amounts were reduced for all lens materials except ANP-1000-OH coated Polymacon buttons, ANP-4000-OH coated polymacon buttons and ANP-hyaluronate coated polymacon buttons. These poor results were all obtained with virgin polymacon materials which appears to react differently than polymacon contact lenses, such as Sofspin lenses. Overall, these in vitro protein deposition studies demonstrated significant to dramatic decreases in protein deposition from artificial tears on various contact lens materials during a one week period.

EP 0 326 579 B1

Table 5: __In Vitro__ Proteins Adsorption from Artificial
Tears: $^3$H Proteins*

| Biocompat-ible Agent | Contact Material | ugHSA/ $cm^2$ | % of control | ugHgg/ $cm^2$ | % of control | ugLys/ $cm^2$ | % of control | ug Total Protein | % of control | % Reduction Total Protein |
|---|---|---|---|---|---|---|---|---|---|---|
| Controls | Polyvinyl Chloride | .525 | 100.pp | .524 | .441 | | 1.49 | | | |
| | Sofspin (Polymacon) | 1.256 | | 1.33 | | .576 | | 3.162 | | |
| | Permaflex | .978 | | 1.953 | 1.866 | | 4.797 | | | |
| | Vistamarc | .553 | | .343 | 47.86 | | 48.76 | | | |
| | Lidofilcon | 2.154 | | 1.009 | 1.423 | | 4.586 | | | |
| | Silicone | 1.121 | | .272 | .264 | | 1.657 | | | |
| | **Polymacon Buttons | 32.23 | | 6.983 | 2.46 | | 41.673 | | | |
| ANP-1000-OH | Polyvinyl Chloride | .298 | 56.8 | .696 | 133 | .0384 | 8.7 | 1.032 | 69.3 | 30.7 |
| | Sofspin | .241 | 19.2 | .191 | 14.4 | .04 | 6.9 | .472 | 14.93 | 85.07 |
| | Permaflex | .582 | 59.5 | 1.351 | 69.2 | 1.693 | 90.73 | 3.626 | 75.6 | 24.4 |
| | Vistamarc | .187 | 33.8 | .378 | 110.2 | 39.19 | 81.88 | 39.76 | 81.5 | 18.5 |
| | Lidofilcon | .640 | 29.7 | .440 | 43.6 | 1.73 | 121.5 | 2.81 | 61.3 | 38.8 |
| | Silicone | .103 | 9.2 | 1.016 | 373.5 | .214 | 81.1 | 1.333 | 80.4 | 19.6 |
| | **Polymacon Buttons | 36.00 | 111.0 | 5.44 | 77.9 | 2.47 | 100.0 | 43.91 | 105.4 | (-5.4) |
| ANP-4000 | Polyvinyl Chloride | .430 | 81.9 | .356 | 67.9 | .148 | 33.6 | .934 | 62.7 | 37.3 |
| | Sofspin | 1.16 | 92.4 | .608 | 45.7 | .297 | 51.6 | 2.065 | 65.3 | 34.7 |
| | Vistamarc | .187 | 33.8 | .686 | 200.0 | 43.24 | 90.3 | 44.11 | 90.5 | 9.5 |
| | Silicone | 1.082 | 96.5 | .242 | 88.9 | .210 | 79.5 | 1.534 | 92.6 | 7.4 |
| | **Polymacon Buttons | 37.62 | 116.7 | 4.172 | 59.7 | 1.863 | 75.7 | 43.655 | 104.7 | (4.7) |

14

| Biocompatible Agent | Contact Material | ugHSA/cm² | % of control | ugHgg/cm² | % of control | ugLys/cm² | % of control | ug Total Protein | % of control | % Reduction Total Protein |
|---|---|---|---|---|---|---|---|---|---|---|
| nBBA-Jeff | Polyvinyl Chloride | .632 | 120.3 | .576 | 109.9 | .072 | 16.33 | 1.28 | 85.9 | 14.1 |
| | Sofspin | .665 | 52.9 | .859 | 64.5 | .531 | .922 | 2.055 | 64.9 | 35.1 |
| | Silicone | .978 | 87.2 | .061 | 22.4 | .068 | 25.8 | 1.107 | 66.8 | 33.2 |
| | **Polymacon Buttons | 26.35 | 81.76 | .01 | .14 | .01 | .41 | 26.37 | 63.28 | 36.72 |
| BBA-Jeff | Polyvinyl Chloride | .326 | 62.1 | .454 | 86.6 | .290 | 66.7 | 1.07 | 71.8 | 28.2 |
| | Silicone | .921 | 82.15 | .089 | 32.7 | .149 | 56.4 | 1.159 | 69.9 | 30.1 |
| | **Polymacon Buttons | 30.61 | 94.9 | 3.695 | 52.9 | .01 | .40 | 34.32 | 82.34 | 17.66 |
| ANP-Jeff | Polyvinyl Chloride | .486 | 92.6 | .456 | 87.0 | .192 | 43.5 | 1.134 | 76.1 | 23.9 |
| | Silicone | .904 | 80.6 | .231 | 83.7 | .257 | 97.6 | 1.392 | 84.01 | 15.99 |
| | **Polymacon Buttons | 35.48 | 110.1 | 5.62 | 80.5 | 1.94 | 78.86 | 43.04 | 103.3 | (3.3) |

\* The values are calculated from 10 replicates
\*\* Polymacon buttons are based on volume rather than surface area cm³
\*\*\* Values in parentheses indicate an increase in protein adsorption

The results of the in vitro cholesterol deposition studies are given in Table 6. The amount of cholesterol deposition after 7 days of incubation in artificial tears was reduced by as much as 83% on polyvinyl chloride. All contact lens types exhibited reduction of cholesterol deposition except the polymacon button pieces. Again, these materials react differently than the contact lenses made of the same polymer.

EP 0 326 579 B1

Table 6

| Cholesterol Adsorption from Artificial Tears: $^3$H Cholesterol | | | | |
|---|---|---|---|---|
| Biocompatible Agent | Contact Material | *ug Cholesterol/cm$^2$ | % of Control | % Reduction |
| Controls | Polyvinyl Chloride | .096 | 100 | 72.9 |
| | Sofspin | .091 | 100 | |
| | Permaflex | .196 | 100 | |
| | Vistamarc | .032 | 100 | |
| | Lidofilcon | .053 | 100 | |
| | Silicone | .103 | 100 | |
| | Polymacon Button | .215 | 100 | |
| ANP-1000 | Polyvinyl Chloride | .026 | 27.1 | 72.9 |
| | Sofspin | .075 | 82.4 | 17.6 |
| | Permaflex | .134 | 68.4 | 31.6 |
| | Vistamarc | .028 | 87.5 | 12.5 |
| | Lidofilcon | .046 | 86.8 | 13.21 |
| | Silicone | .086 | 83.5 | 16.5 |
| | Polymacon Button | .245 | 113.9 | **(13.9) |
| ANP-4000 | Polyvinyl Chloride | .0166 | 17.3 | 82.7 |
| | Sofspin | .101 | 110.9 | (10.9) |
| | Permaflex | .134 | 68.4 | 31.6 |
| | Vistamarc | .023 | 71.9 | 28.1 |
| | Silicone | .104 | 100.9 | (0.9) |
| | Polymacon Buttom | .248 | 115.3 | (15.3) |
| nBBA-Jeff | Polyvinyl Chloride | .038 | 39.6 | 60.4 |
| | Sofspin | .138 | 151.6 | (51.6) |
| | Permaflex | .164 | 83.7 | 16.33 |
| | Silicone | .072 | 69.9 | 30.1 |
| | Polymacon Button | .148 | 68.8 | 31.2 |
| BBA-Jeff | Polyvinyl Chloride | .0214 | 22.3 | 77.7 |
| | Silicone | .107 | 103.8 | (3.8) |
| | Polymacon Button | .230 | 106.9 | (6.9) |
| ANP-Jeff | Polyvinyl Chloride | .028 | 29.2 | 70.2 |
| ANP-Hyaluronate | Silicone | .224 | 217.5 | (117.5) |
| | Polymacon Button | .238 | 110.6 | (10.6) |

* Values were the averages of ten replicates.
** Values in parentheses indicate increase in cholesterol adsorption.

C. Amino Acid Analysis. Control and surface modified lenses were incubated in the artificial tear solution for one week at 37°C with gentle agitation. The lenses were washed with 5 10ml washes of 0.85% NaCl, then hydrolyzed with 6N HCl and the hydrolysates subjected to standard amino acid analyses on an amino acid analyzer. Total amino acid content of control and surface modified lenses were compared to each other. Reduction in total amino acid content indicated a reduction in protein absorption.

The total amino acid analyses of the acid hydrolyzed contact lenses are given in Table 8. These results are expressed as total amino acids in nmole/ml. These results again indicated that the ANP-1000-OH, ANP-4000-OH and nBBA-Jeff modifications of Sofspin polymacon lenses reduced the deposition of proteins on the lenses after 7 days of incubation in artificial human tears.

16

Table 7

| Total Amino Acid Analyses from Artificial Tear Deposits on Contact Lenses | | | |
|---|---|---|---|
| Contact Material | Biocompatible Agent | Total Amino Acids nmol/lens | % Reduction |
| Sofspin | ANP-1000 | 62.75 | 59.7 |
| | ANP-4000 | 136.272 | 12.4 |
| | nBBA-Jeff | 105.381 | 32.3 |
| | Control | 155.616 | ---- |
| Permalens | ANP-1000 | 168.714 | 32.5 |
| | ANP-4000 | 210.207 | 15.9 |
| | nBBA-Jeff | 181.302 | 27.5 |
| | Control | 249.939 | ---- |

4. In Vitro Toxicity Testing. The biocompatible lens materials described above were tested for irritant or toxicity responses. Control and biocompatible lenses were prepared under sterile conditions with final washing procedures conducted in a laminar flow hood. The lenses were placed in a solution of a known high glucose cell medium such as Dulbecco's Modified Essential Medium, with 10% fetal calf serum, and 5% glutamine (to which antibiotic and anti-fungal agents had been added). Pieces of viable 3-5 month fetal bovine corneas were placed on top of the lenses. The epithelial surface of the cornea was placed in contact with the lens surface in some studies, and the endothelial cell surface was placed directly on the lens surface in other studies. The systems were placed in culture at 7-10% $CO_2$ and 37°C for 1-2 week periods. At various intervals after initiation of culture the viabilities of the epithelial and/or endothelial cells were assessed by staining procedures.

5. In Vitro Assay for Stability of Covalent Bond. The stability of the covalent attachment was assessed by subjecting the surface-modified polymacon (polymethacrylate) to enzymatic cleaner (papain), thermal disinfection and chemical disinfection (buffered acqueous solution containing sodium chloride, sodium-borate and boric acid.) These results are given in Table 8.

17

Table 8

| Stability of Covalent Linkage to Cleaning Disinfection Procedures | | | |
|---|---|---|---|
| Biocompatible Agent | Treatment | pmole/cm3 | % Remaining |
| ANP-1000 | No treatment<br>Enzymatic cleaner<br>Boiling<br>Chemical cleaner | 831<br>862<br>761<br>721 | 100<br>100<br>91.6<br>86.8 |
| ANP-4000 | No treatment<br>Enzymatic cleaner<br>Boiling<br>Chemical cleaner | 1574<br>2012<br>2092<br>1564 | 100<br>100<br>100<br>99.4 |
| nBBA-2000 | No treatment<br>Enzymatic cleaner<br>Boiling<br>Chemical cleaner | 1732<br>1785<br>1795<br>1129 | 100<br>100<br>100<br>65.2 |
| BBA-2000 | No treatment<br>Enzymatic cleaner<br>Boiling<br>Chemical cleaner | 1651<br>1996<br>1619<br>1409 | 100<br>100<br>98.1<br>85.3 |
| ANP-Hyaluronate | No treatment<br>Enzymatic cleaner<br>Boiling<br>Chemical Cleaner | 300<br>317<br>340<br>307 | 100<br>100<br>100<br>100 |

\* Values are the averages of 10 replicates

The covalent linkages remained 100% stable to enzymatic cleaning and thermal disinfection. There was some loss of biocompatible agent with the chemical disinfection procedures except for the ANP-hyaluronate.

6. In Vivo Assessment of Biocompatibility. Preliminary in vivo biocompatibility testing was conducted in rabbit model systems. A population of at least 24 animals was used in each study. Scleral lenses designed to fit rabbit eyes were used in these studies. The rabbits wore lenses according to the following schedule:

6 rabbits     No lens left eye, control lens right eye

6 rabbits     Control lens left eye; physically treated lens (same physical treatment as surface modified lenses but not coated with biocompatible agent) right eye

12 rabbits     Control lens left eye; surface-modified lens right eye.

The rabbits were anesthetized with Ketamine/Xylazine prior to placing the lenses in the rabbit eyes. A methyl cellulose/normal saline wetting solution was applied hourly to maintain adequate eye and lens lubrication. The contacts were worn 8 hours/day. After chosen periods of lens wear the rabbits were analyzed by slit lamp and fluorescein dye methods. The degree of eye irritation was graded by the McDonald-Shadduck scale as described in T.O. McDonald and J.A. Shadduck, "Eye Irritation," in Advances in Modern Toxicology, Vol. 4, pp. 162-166 (1977) incorporated herein by reference. The McDonald-Shadduck procedure allows the investigator to grade conjuctival congestion, conjuctival swelling, conjunctival discharge, aqueous flare, iris involvement and corneal cloudiness and other characters on a scale of 0-4, with 0 being normal and +4 the most complete involvement.

The results of the in vivo rabbit studies are given in Table 9. The McDonald-Shadduck scores for the rabbits are represented in the table. Mann-Whitney U tests were performed on these results and indicated that there were no statistical differences between the surface-modified and control lenses ($p < 0.05$). Therefore, these tests indicate that in this 4-day study in rabbits, no detectable differences in conjunctival congestion, conjunctival swelling, conjuntival discharge, aqueous flare, iris involvement, corneal cloudiness, pannus vascularization, norepithelial damage of the surface modified lenses was detected as compared to control lenses. The irritation which was demonstrated in the control and the modified lenses appeared to be associated with the rabbits development of tolerance to contact lenses.

Table 9

| Average McDonald-Shadduck Scores For In Vivo Rabbit Study | | | | |
|---|---|---|---|---|
| | Day 1 | Day 2 | Day 3 | Day 4 |
| Control Lens | 0.53 | 0.78 | 0.19 | 0.33 |
| Surface Modified Lens | 0.58 | 0.65 | 0.17 | 0.21 |

Example 3

Coupling of Albumin, Heparin and Urokinase to Polyurethane Tubing

1. Preparation of Photolabeled Albumin. Serum albumin (canine) was dissolved in 0.1M borate buffer at pH 9.0. A volume of 4-azido-2-nitrophenyl-6-aminocaproyl-N-oxysuccinimide (ANP-EAC-NOS) solution at 25 mg/ml in DMF to provide a 10 fold molar excess of ANP-EAC-NOS over albumin was added to the albumin with stirring at room temperature in the dark over about 12 hours using a syringe drive. The solution was then dialyzed in the dark against three one liter volumes of phosphate buffered saline (PBS). After dialysis, the solution was centrifuged to remove insoluble material. The absorance at 470 nm of a 1/100 dilution was measured spectrophotometrically to estimate the ANP/albumin ratio.

2. Photocoupling of Albumin to Polyurethane Tubing. Polyurethane tubing was dipped into dioxane for thirty seconds after which it was immediately rinsed with deionized water. The etched tubing was immersed in a solution of photoreactive albumin for at least two hours at room temperature in the dark with mixing. The tubing was then air dried for at least ten minutes in the dark, then exposed to high intensity visible light for at least one hour. The immersion in photoreactive albumin, drying and photolyzing was repeated twice, the last time the dipping was left overnight. The tubing was then washed in 1.0 N NaCl for one hour at room temperature. The NaCl solution was changed at least once during the one hour.

3. Coupling of Heparin to Albumin on Polyurethane Tubing. The albumin-polyurethane tubing was immersed in 5.0 ml. of deionized water. 200 mg of 1-ethyl-3-(3-dimethylaminopropyl) carbodimide (EDC) was dissolved in the water, then the pH was adjusted to 4.0 with 1N HCl. One ml of water containing 30 mg of heparin was added to the EDC solution. The pH was again adjusted to 4.0 with 1N HCl. The solution containing the immersed polyurethane tubing was mixed for two hours at room temperature after which another 200 mg of EDC was added. The reaction was then allowed to continue overnight at room temperature. The tubing was then rinsed in PBS to remove uncoupled heparin and reaction by-products.

4. Coupling of Urokinase to Albumin on Polyurethane Tubing. Polyurethane tubing having serum albumin immobilized thereon was immersed in 1.25% glutaraldehyde in 0.1M phosphate buffer, pH 7.0 for 15-18 hours at room temperature. The tubing was then washed in deionized water for 30 minutes, the water being changed at least once during that time. The polyurethane-albumin-glutaraldehyde was then immersed in a solution of urokinase (8.3 units/ml, 2-3 mg/ml) in 0.1M borate of pH 9.0 and mixed overnight at 4°C. The tubing was then rinsed for four hours in PBS after which it was assayed for urokinase activity.

Two polymers were used as the solid surface, polyurethane and polyhema. The modified surfaces yielded 1-10 mg heparin/cm$^2$ an 0.6-1.3 mg urokinase/cm$^2$.

Example 4

Coupling of a Film to a Solid Surface

Formation of a Coating Film and its Covalent Attachment to a Surface. Preparation of ANP-hyaluronic acid. Photolabeled derivatives of hyaluronic acid (ANP-EAC-Jeffamine, BBA-Jeffamine and nitroBBA-Jeffamine) were prepared as previously described.

Films are formed from the photoreactive coating material and placed on surfaces of contact lenses (by dipping and drying) in the dark. Covalent attachment to the biomaterial surface and strengthening of the film by intermolecular cross-linking may be accomplished through illumination.

In another example, an artificial hip joint is soaked in ANP-EAC-Jeffamine-hyaluronic acid (.1:1 mg/ml) for three hours in the dark. The joint is then removed from solution and allowed to dry forming a thin film of coating material on the artificial joint. The film is then covalently attached to the joint by illumination at 400 to 450 nm for 8 hrs. at 4°C. The joint is then rinsed in physiological saline to remove uncoupled ANP-EAC-Jeffamine-hyaluronate. The hyaluronic acid bound to the bone reduces friction and reduces wearing of the bone in the joint area.

100 mg of bovine serum albumin is dissolved in 2 ml of .1M borate buffer pH 9. 14mg of ANP-AUD-NOS is dissolved in 50 ul of dimethylformamide (DMF). The ANP-AUD-NOS solution is added to the BSA solution slowly over 15 hrs at room temperature in the dark with good stirring. After an additional 3 to 4 hours of stirring the solution is dialyzed against .1M borate buffer at pH 9 in the dark over 24 hrs. with at least 4 changes of 2 liters each of buffer. The dialyzed ANP-BSA is pipetted onto paraffin films in 100 ul aliquots and dried in the dark. After the films have dried they are overlaid with 100 $\mu$g aliquots of 1.25% glutaraldehyde and borate buffer pH 9 and incubated for one hour at room temperature in the dark. The films are then washed by slowly dropping water onto them while still on the paraffin films and allowing the water to run off. After one hour of such washing, the films are redried and then carefully lifted off the paraffin film and transferred to a plastic surface (e.g., polyurethane). On the plastic surface the films are again wetted with water containing 20% dioxane, then redried in the dark. The films on the plastic surfaces are then exposed to high intensity visible light for four hours at 4°C to bind the film to the surface with a fan blowing across the surface to prevent excessive heating of the surface.

**Claims**

1. A method of producing a biocompatible device from a biomaterial substrate having a solid surface, which comprises:

   providing the solid surface with a biocompatible surface comprising a biocompatible agent by use of a chemical-linking moiety possessing a photochemically reactive group and possessing a different reactive group;

   characterised in that:

   in the chemical-linking moiety one of said groups is unresponsive to activation by a stimulus to which the other group is responsive;

   applying stimulus to sequentially activate said groups to covalently bond said different reactive group to the molecules of the biocompatible agent and to photochemically covalently bond the linking moiety to the solid surface with a sufficient population density to enable the molecules of the biocompatible agent to effectively shield the solid surface with the biocompatible surface;

   wherein the chemical linking moiety has the formula A-X-B in which:

   A represents a photochemically reactive group capable, in response to specific activation, of bonding covalently to the solid surface;

   B represents a different reactive group capable in response to specific activation to which group A is unresponsive, of forming a covalent bond to the biocompatible agent; and

   X represents a relatively inert, non-interfering skeletal moiety joining groups "A", and "B", the skeletal moiety being resistant to cleavage in aqueous physiological fluids.

2. A method according to Claim 1, wherein said different reactive group is a thermochemically reactive group.

3. A method according to Claim 1, wherein said different reactive group is a photochemically reactive group.

4. A method according to Claim 1, 2 or 3, wherein the stimulus to activate said photochemically-reactive group is applied subsequent to the application of the stimulus to activate said different reactive group.

5. A method according to any of the preceding claims, wherein the solid surface is substantially thermochemically unreactive.

6. A method according to Claim 1, comprising the steps of:

   providing a chemical linking moiety comprising a photochemically reactive group capable upon activation of covalently bonding to said solid surface, and a different reactive group, capable of binding to the molecules of the biocompatible agent, one of the groups being unresponsive to a stimulus to which the other group responds;

   activating said different reactive group of the chemical linking moiety in the presence of molecules of the biocompatible agent to cause said different reactive group to covalently bind the chemical linking moiety to said molecules; and

activating the photochemically reactive group of the chemical linking moiety in the presence of the solid surface of the biomaterial to cause the photochemical reactive group to covalently bond to the solid surface in a sufficient population density to provide a biocompatible effective surface.

7. A method according to Claim 1, wherein X is a $C_1$ - $C_{10}$ alkyl group.

8. A method according to Claim 1, wherein group A is taken from: aryl, alkyl and acyl azides; alkyl and 2-ketodiazo derivatives and diazirines (carbene generators); aromatic ketones (triplet oxygen generators); aromatic diazonium derivatives and carbonium ion and radical generators.

9. A method according to Claim 8, wherein group A is taken from: azidonitrophenyls, fluoroazido nitrobenzenes and aromatic ketones.

10. A method according to Claim 1, wherein group B is a thermochemically reactive group taken from: nitrophenylhalides, alkylamino, alkylcarboxyl, alkylthiol, alkylaldehyde, alkylmethylimidate, alkylisocyanate, alkylisothiocyanate and alkylhalide groups.

11. A method according to Claim 1, wherein group B is taken from: carboxyl groups, hydroxyl groups, primary amino groups, thiol groups, maleimides and halide groups.

12. A biocompatible device which comprises:

a substrate of a biomaterial having a solid surface and carrying molecules of a biocompatible agent;

a chemical linking moiety residue binding individual molecules of the biocompatible agent to the solid surface;

characterised in that

the chemical linking moiety residue includes a residue of a photochemically reactive group covalently bonded to the solid surface, and includes a residue of a different reactive group covalently bonded to molecules of the biocompatible agent, the photochemically reactive group residue being bonded to the solid surface so that the individual molecules of the biocompatible agent are positioned sufficiently proximate to one another as to cause said molecules to effectively shield the solid surface and to provide a biocompatible effective surface, wherein the chemical linking moiety has the formula A-X-B in which:

A represents a photochemically reactive group capable, in response to specific activation, of bonding covalently to the solid surface;

B represents a different reactive group capable ,in response to specific activation, to which group A is unresponsive, of forming a covalent bond to the biocompatible agent; and

X represents a relatively inert, non-interfering skeletal moiety joining groups "A", and "B", the skeletal moiety being resistant to cleavage in aqueous physiological fluids.

13. A biocompatible device according to Claim 12, wherein said device is suitable for implantation in a body.

14. A device according to Claim 12, wherein the solid surface is substantially thermochemically unreactive.

15. A device according to Claim 12 wherein the biocompatible agent is a cell attachment factor.

16. A device according to Claim 15, wherein the cell attachment factor is laminin or fibronectin.

17. A device according to Claim 12, wherein the biocompatible agent is a growth factor.

18. A device according to Claim 17, wherein the growth factor is endothelial growth factor, epithelial growth factor, osteoblast growth factor, fibroblast growth factor, platelet derived growth factor, neural growth factor, or angiogenin growth factor.

19. A device according to Claims 12, wherein the biocompatible agent is collagen.

**20.** A device according to Claims 12, wherein the biocompatible agent is albumin.

**21.** A device according to Claim 12, wherein the biocompatible agent is a hydrophilic polymer.

**22.** A device according to Claim 21, wherein the hydrophilic polymer is polyethylene glycol, hyaluronic acid, methyl cellulose, collagen, chondroitin sulfate or chitosan.

**23.** A device according to any of Claims 12 to 22, wherein the solid surface is vascular graft tubing, dialysis tubing or membrane, blood oxygenator tubing or membrane, ultrafiltration membrane, a blood bag, a catheter, an intraaortic balloon, a suture, a soft or hard tissue prosthesis, a synthetic prosthesis, an artificial organ, or a lens for the eye.

**24.** A device according to Claim 12, wherein said molecules of the biocompatible agent are joined to one another to form a biocompatible film.

**25.** A device according to Claim 12, wherein the biocompatible agent is an anti-microbial agent.

**26.** A device according to Claim 25, wherein the biocompatible agent is lysozyme or penicillin.

**27.** A device according to Claim 12, wherein the biocompatible agent is an anti-thrombogenic agent.

**28.** A device according to Claim 27, wherein the anti-thrombogenic agent is heparin, streptokinase, tissue plasminogen activator or urokinase.

**29.** A device according to Claim 12, wherein the group X of the biocompatible agent is a polyethylene glycol or a polypeptide.

**30.** A device according to Claim 12, wherein the biocompatible agent is a fatty acid.

**31.** A device according to any of Claims 12 to 30, wherein the solid surface is a polymeric substrate.

**32.** A device according to Claim 31 wherein the polymeric substrate is polyurethane, polyvinylpyrrolidone, polyamide, polyamine, poly-tetrafluoroethylene, poly ($\underline{p}$-phenyleneterephthalamide), polyvinyl chloride, polypropylene, polyacrylate, polymethacrylate, polyacrylamide, polyimine, polyester, polyethylene, cellulose, or silicone.

**33.** A device according to Claim 23, wherein the lens for the eye is a contact lens or an intraocular lens.

**34.** A biocompatible device according to Claim 12, wherein the device is a cell culture device having a solid surface carrying molecules of a biocompatible agent selected from the group consisting of growth factors, cell attachment factors, synthetic polymers, antithrombogenic agents, carbohydrates, fatty acids, and antimicrobial agents, and a chemical linking moiety residue covalently binding individual molecules of the biocompatible agent to the solid surface, the chemical linking moiety residue including a residue of a photochemically reactive group covalently bonded to the solid surface, and a residue of a different reactive group covalently bonded to molecules of the biocompatible agent, one of the groups being unresponsive to a stimulus to which the other group responds, the photochemically reactive group residue being bonded to the solid surface so that individual molecules of the biocompatible agent are positioned sufficiently proximate to one another as to cause said molecules to effectively shield the solid surface and to provide a biocompatible effective surface.

**35.** A cell culture device according to Claim 34, wherein the device is provided in a form selected from the group consisting of plates, sheets, and tubes.

**36.** A cell culture device according to Claim 34, wherein the device provides a polymeric surface for cell attachment selected from the group consisting of polyvinyl chloride, polyethylene, polypropylene, polyfluorotetraethylene, silicone elastomer, polyurethane, polystyrene, and polyacrylates, including polymethacrylates.

22

37. A cell culture device according to Claim 34, wherein the biocompatible agent is a growth factor selected from the group consisting of fibronectin, laminin, collagen, endothelial growth factor, and human serum albumin.

38. A cell culture device according to Claim 34, wherein the synthetic polymer is a hydrophilic synthetic polymer.

39. A method according to Claim 1, wherein X is: a C1-10 alkyl group such as polymethylene; a carbohydrate such as polymethylol; a polyoxyethylene such as polyethylene glycol; or a polypeptide such as polylysine.

40. A device according to Claim 12, wherein group A is taken from: aryl, alkyl, acyl azides; alkyl and 2-ketodiazo derivatives and diazirines (carbene generators); aromatic ketones (triplet oxygen generators); aromatic derivatives and carbonium ion and radical generators.

41. A device according to Claim 40, wherein group A is taken from: azidonitrophenyls, fluoroazidonitrobenzenes, and aromatic ketones.

42. A device according to Claim 12, wherein group B is a thermochemically reactive group taken from: nitrophenylhalides, alkylamino, alkylcarboxyl, alkylthio, alkylaldehyde, alkylmethylimidate, alkylisocyanate, alkylisothiocyanate, and alkylhalide groups.

43. A device according to Claim 12, wherein group B is taken from: carboxyl groups, hydroxyl groups, primary amino groups, thiol groups, maleimides, and halide groups.

44. A device according to Claim 12, wherein X is a C1-C10 alkyl group.

45. A device according to Claim 12, wherein X is: a C1 - 10 alkyl group such as polymethylene; a carbohydrate such as polymethylol; a polyoxyethylene such as polyethylene glycol; or a polypeptide such as polylysine.

46. A device according to Claim 29, wherein the polypeptide is polylysine.

**Patentansprüche**

1. Verfahren zur Herstellung einer biokompatiblen Vorrichtung aus einem Biomaterialsubstrat mit fester Oberfläche, wobei:
die feste Oberfläche durch Verwendung eines chemisch verbindenden Anteils, der eine fotochemisch reaktive Gruppe und eine andere reaktive Gruppe aufweist, mit einer biokompatiblen Oberfläche versehen wird, die ein biokompatibles Agens enthält;
**dadurch gekennzeichnet,**
daß bei dem chemisch verbindenden Anteil eine der Gruppen auf eine Aktivierung durch einen Reiz nicht reagiert, auf welche die andere Gruppe reagiert;
daß ein Reiz aufgebracht wird, um nacheinander die Gruppen zu aktivieren, um die unterschiedlichen reaktiven Gruppen kovalent an die Moleküle des biokompatiblen Agens zu binden und um den verbindenden Anteil mit einer ausreichenden Populationsdichte fotochemisch kovalent an die feste Oberfläche zu binden, um die Moleküle des biokompatiblen Agens in die Lage zu versetzen, die feste Oberfläche mit der biokompatiblen Oberfläche wirksam abzuschirmen;
wobei der chemische verbindende Anteil die Formel A-X-B aufweist, in welcher:

A für eine fotochemisch reaktive Gruppe steht, die sich in Reaktion auf eine spezifische Aktivierung kovalent an die feste Oberfläche binden kann;

B für eine andere reaktive Gruppe steht, die in Reaktion auf eine spezifische Aktivierung, auf welche die Gruppe A nicht reagiert, eine kovalente Bindung zu dem biokompatiblen Agens bilden kann; und

X für einen relativ inerten, keinen Einfluß ausübenden Skelettanteil steht, der die Gruppen "A" und "B" verbindet, wobei der Skelettanteil gegenüber einer Spaltung in wässrigen physiologischen Fluiden widerstandsfähig ist.

EP 0 326 579 B1

**2.** Verfahren nach Anspruch 1, wobei die andere reaktive Gruppe eine thermochemisch reaktive Gruppe ist.

**3.** Verfahren nach Anspruch 1, wobei die andere reaktive Gruppe eine fotochemisch reaktive Gruppe ist.

**4.** Verfahren nach Anspruch 1, 2 oder 3, wobei die Anregung zur Aktivierung der fotochemisch reaktiven Gruppen anschließend an die Aufbringung der Anregung zur Aktivierung der anderen reaktiven Gruppe aufgebracht wird.

**5.** Verfahren nach einem der vorhergehenden Ansprüche, wobei die feste Oberfläche im wesentlichen thermochemisch inreaktiv ist.

**6.** Verfahren nach Anspruch 1 mit folgenden Schritten:
Vorsehen eines chemisch verbindenden Anteils, der eine fotochemisch reaktive Gruppe aufweist, die in der Lage ist, sich bei Aktivierung kovalent an die feste Oberfläche zu binden, sowie eine andere reaktive Gruppe, die in der Lage ist, sich an die Moleküle des biokompatiblen Agens zu binden, wobei eine der Gruppen nicht auf einen Reiz anspricht, auf welchen die andere Gruppe anspricht;
Aktivierung der anderen reaktiven Gruppe des chemisch verbindenden Anteils bei Gegenwart von Molekülen des biokompatiblen Agens, um zu bewirken, daß sich die andere reaktive Gruppe im chemisch verbindenden Anteil kovalent an die Moleküle bindet; und
Aktivieren der fotochemisch reaktiven Gruppe des chemisch verbindenden Anteils bei Gegenwart der festen Oberfläche des Biomaterials, um zu bewirken, daß die fotochemisch reaktive Gruppe sich in einer ausreichenden Populationsdichte kovalent an die feste Oberfläche bindet, um eine biokompatible wirksame Oberfläche zu schaffen.

**7.** Verfahren nach Anspruch 1, wobei X eine $C_1$-$C_{10}$ Alkylgruppe ist.

**8.** Verfahren nach Anspruch 1, wobei die Gruppe A aus folgender Gruppe ausgewählt wird: Aryl-, Alkyl- und Akylsäuren; Alkyl- und 2-Ketodiazo-Derivate und Diazyrine (Karbengeneratoren); aromatische Ketone (Triplett Sauerstoffgeneratoren), aromatische Diazoniumderivate und Karbonium-Ion und Radikalgeneratoren.

**9.** Verfahren nach Anspruch 8, wobei die Gruppe A aus folgender Gruppe ausgewählt wird: Azidonitrophenyle, Fluoroazidonitrobenzine und aromatische Ketone.

**10.** Verfahren nach Anspruch 1, wobei die Gruppe B eine thermochemisch reaktive Gruppe ist, die aus folgender Gruppe ausgewählt ist:
Nitrophenylhalogenide, Alkylamino-, Alkylcarboxyl-, Alkylthiol-, Alkylaldehyd-, Alkylmethylimidat-, Alkylisozyanat-, Alkylisothiozyanat- und Alkylhalogenidgruppen.

**11.** Verfahren nach Anspruch 1, wobei die Gruppe B aus folgender Gruppe ausgewählt wird: Carboxyl-Gruppen, Hydroxyl-Gruppen, primäre Amino-Gruppen, Thiol-Gruppen, Maleimide und Halogenidgruppen.

**12.** Biokompatible Vorrichtung mit:
einem Substrat aus einem Biomaterial, das eine feste Oberfäche aufweist, und Moleküle eines biokompatiblen Agens trägt;
einem chemisch verbindenden Anteilsrückstand, der einzelne Moleküle des biokompatiblen Agens an die feste Oberfläche bindet;
**dadurch gekennzeichnet**,
daß der chemisch verbindende Anteilsrückstand einen Rest einer fotochemisch reaktiven Gruppe umfaßt, die kovalent an die feste Oberfläche gebunden ist, und einen Rest einer anderen reaktiven Gruppe umfaßt, die kovalent an die Moleküle des biokompatiblen Agens gebunden ist, wobei der Rest der fotochemisch reaktiven Gruppe so an die feste Oberfläche gebunden ist, daß die einzelnen Moleküle des biokompatiblen Agens ausreichend nahe beieinander angeordnet sind, um zu bewirken, daß die Moleküle die feste Oberläche wirksam abschirmen, und um eine biokompatibel wirksame Oberfläche zu schaffen, wobei der chemisch verbindende Anteil die Formel A-X-B aufweist, in welcher
A für eine fotochemisch reaktive Gruppe steht, die in der Lage ist, sich in Reaktion auf eine

24

spezifische Anregung kovalent an die feste Oberfläche zu binden;

B für eine andere reaktive Gruppe steht, die in der Lage ist, in Reaktion auf eine spezifische Aktivierung, auf welche die Gruppe A nicht reagiert, eine kovalente Bindung zu dem biokompatiblen Agens zu bilden; und

X für einen relativ inerten, keinen Einfluß ausübenden Skelettanteil steht, der die Gruppen "A" und "B" verbindet, wobei der Skelettanteil einer Spaltung in wässrigen physiologischen Fluiden widersteht.

13. Biokompatible Vorrichtung nach Anspruch 12, wobei die Vorrichtung dazu geeignet ist, in einen Körper implantiert zu werden.

14. Vorrichtung nach Anspruch 12, wobei die feste Oberfläche im wesentlichen thermochemisch inreaktiv ist.

15. Vorrichtung nach Anspruch 12, wobei das biokompatible Agens ein Zellbefestigungsfaktor ist.

16. Vorrichtung nach Anspruch 15, wobei der Zellbefestigungsfaktor Laminin oder Fibronektin ist.

17. Vorrichtung nach Anspruch 12, wobei das biokompatible Agens ein Wachstumsfaktor ist.

18. Vorrichtung nach Anspruch 17, wobei der Wachstumsfaktor ein Endothel-Wachstumsfaktor, ein Epithel-Wachstumsfaktor, ein Osteoplast-Wachstumsfaktor, ein Fibroplast-Wachstumsfaktor, ein Plättchen abgeleiteter Wachstumsfaktor, ein neuraler Wachstumsfaktor oder ein Angeogenin-Wachstumsfaktor ist.

19. Vorrichtung nach Anspruch 12, wobei das biokompatible Agens Kollagen ist.

20. Vorrichtung nach Anspruch 12, wobei das biokompatible Agens Albumin ist.

21. Vorrichtung nach Anspruch 12, wobei das biokompatible Agens ein hydrophiles Polymer ist.

22. Vorrichtung nach Anspruch 21, wobei das hydrophile Polymer Polyethylenglykol, Hyaluronsäure, Methylzellulose, Kollagen, Chondroitinsulfat oder Chitosan ist.

23. Vorrichtung nach einem der Ansprüche 12 bis 22, wobei die feste Oberfläche eine Vaskulargraftrohr, ein Dialyserohr oder eine -membran, ein Blut mit Sauerstoff anreicherndes Rohr oder eine solche Membran, eine Ultrafiltrationsmembran, eine Bluttasche, ein Katheter, ein intraaortischer Ballon, eine Naht, eine weiche oder harte Gewebeprothese, eine synthetische Prothese, ein künstliches Organ oder eine Augenlinse ist.

24. Vorrichtung nach Anspruch 12, wobei die Moleküle des biokompatiblen Agens miteinander verbunden sind, um einen biokompatiblen Film zu bilden.

25. Vorrichtung nach Anspruch 12, wobei das biokompatible Agens ein Antimikrobenagens ist.

26. Vorrichtung nach Anspruch 25, wobei das biokompatible Agens Lysozym oder Penicillin ist.

27. Vorrichtung nach Anspruch 12, wobei das biokompatible Agens ein Anti-thromboseerzeugendes Mittel ist.

28. Vorrichtung nach Anspruch 27, wobei das Anti-thromboseerzeugende Mittel Heparin, Streptokinase, Gewebeplasminogen-Aktivator oder Urokinase ist.

29. Vorrichtung nach Anspruch 12, wobei die Gruppe X des biokompatiblen Agens ein Polyethylenglykol oder ein Polypeptid ist.

30. Vorrichtung nach Anspruch 12, wobei das biokompatible Agens eine Fettsäure ist.

**31.** Vorrichtung nach einem der Ansprüche 12 bis 30, wobei die feste Oberfläche ein polymeres Substrat ist.

**32.** Vorrichtung nach Anspruch 31, wobei das polymere Substrat Polyurethan, Polyvinylpyrolidon, Polyamid, Polyamin, Polytetrafluoroethylen, Poly-(p-phenylenterephtalamid), Polyvinilchlorid, Polypropylen, Polyacrylat, Pholymetacrylat, Polyacrylamid, Polyimin, Polyester, Polyethylen, Cellulose oder Silikon ist.

**33.** Vorrichtung nach Anspruch 23, wobei die Linse für das Auge eine Kontaktlinse oder eine intraokulare Linse ist.

**34.** Biokompatible Vorrichtung nach Anspruch 12, wobei die Vorrichtung eine Zellkulturvorrichtung mit einer festen Oberfläche ist, die Moleküle eines biokompatiblen Agens trägt, welches aus der Gruppe ausgewählt ist, die besteht aus: Wachstumsfaktoren, Zellbefestigungsfaktoren, synthetischen Polymeren, antithrombogenen Mitteln, Kohlenhydraten, Fettsäuren und Antimikrobenmitteln, sowie einen chemisch verbindenden Anteilsrest, der die einzelnen Moleküle des biokompatiblen Agens kovalent an die feste Oberfläche bindet, wobei der chemisch verbindende Anteilsrückstand einen Rest einer fotochemisch reaktiven Gruppe umfaßt, die kovalent an die feste Oberfläche gebunden ist, und einen Rest einer anderen reaktiven Gruppe, die kovalent an die Moleküle des biokompatiblen Agens gebunden ist, wobei eine der Gruppen auf einen Reiz nicht reagiert, auf welchen die andere Gruppe reagiert, wobei der Rest der fotochemisch reaktiven Gruppe so an die feste Oberfläche gebunden ist, daß einzelne Moleküle des biokompatiblen Agens ausreichend nahe beieinander angeordnet sind, um zu bewirken, daß die Moleküle die Oberfläche wirksam abschirmen, und um eine biokompatibel wirksame Oberfläche zu bilden.

**35.** Zellkulturvorrichtung nach Anspruch 34, wobei die Vorrichtung in einer Form vorgesehen ist, die aus der aus Platten, Blättern und Rohren bestehenden Gruppe ausgewählt ist.

**36.** Zellkulturvorrichtung nach Anspruch 34, wobei die Vorrichtung eine polymere Oberfläche zur Zellbefestigung aufweist, die aus der Gruppe ausgewählt ist, die aus Polyvinylchlorid, Polyethylen, Polypropylen, Polyfluortetraethylen, Silikonelastomer, Polyuretan, Polystyrol und Polyacrylaten, einschließlich Polymetacrylaten besteht.

**37.** Zellkulturvorrichtung nach Anspruch 34, wobei das biokompatible Agens ein Wachstumsfaktor ist, der aus der Gruppe ausgewählt ist, die aus Fibronectin, Laminin, Kollagen, Endothel-Wachstumsfaktor und Humanserumalbumin besteht.

**38.** Zellkulturvorrichtung nach Anspruch 34, wobei das synthetische Polymer ein hydrophiles synthetisches Polymer ist.

**39.** Verfahren nach Anspruch 1, wobei X eine C1-10 Alkylgruppe wie Polymethylen, ein Kohlenhydrat wie Polymethylol, ein Polyoxyethylen wie Polyethylenglykol oder ein Polypeptid wie Polylysin ist.

**40.** Vorrichtung nach Anspruch 12, wobei die Gruppe A aus der Gruppe entnommen wird, die aus Aryl-, Alkyl-, Akylsäuren; Alkyl- und 2 ketodiazo-Derivaten und Diazyrinen (Karbengeneratoren); aromatischen Ketonen (Triplett Sauerstoffgeneratoren); aromatischen Derivaten und Karbonium-Ion und radikalen Generatoren besteht.

**41.** Vorrichtung nach Anspruch 40, wobei die Gruppe A aus Azidonitrophenylen, Fluoroazidonitrobenzinen und aromatischen Ketonen ausgewählt wird.

**42.** Vorrichtung nach Anspruch 12, wobei die Gruppe B eine thermochemisch reaktive Gruppe ist, die aus Nitrophenylhalogeniden, Alkylamino-, Alkylcarboxyl-, Alkylthio-, Alkylaldehyd-, Alkylmethylimidat-, Alkylisozyanat-,Alkylisothiozyanat- und Alkylhalogenidgruppen besteht.

**43.** Vorrichtung nach Anspruch 12, wobei die Gruppe B aus Carboxylgruppen, Hydroxylgruppen, primären Aminogruppen, Thiolgruppen, Maleimiden und Halogenidgruppen ausgewählt wird.

**44.** Vorrichtung nach Anspruch 12, wobei X ein C1-C10 -Alkyl-gruppe ist.

**45.** Vorrichtung nach Anspruch 12, wobei X eine C1-10 -Alkyl-gruppe wie Polymethylen; ein Kohlenhydrat wie Polymethylol; ein Polyoxyethylen wie Polyethylenglykol oder ein Polypeptid wie Polylysin ist.

**46.** Vorrichtung nach Anspruch 29, wobei das Polypeptid Polylysin ist.

**Revendications**

**1.** Procédé de fabrication d'un dispositif biocompatible à partir d'un substrat de biomatériau ayant une surface solide, lequel comprend :
   - l'opération consistant à doter la surface solide d'une surface biocompatible comprenant un agent biocompatible par utilisation d'un fragment de liaison chimique possédant un groupe photochimiquement réactif et possédant un groupe réactif différent ;

   caractérisé par le fait que :
   - dans le fragment de liaison chimique l'un desdits groupes est non sensible à l'activation par un stimulus auquel l'autre groupe est sensible ;
   - l'application d'un stimulus pour activer séquentiellement lesdits groupes pour lier de façon covalente ledit groupe réactif différent aux molécules de l'agent biocompatible et pour lier par voie photochimique, de façon covalente, le fragment de liaison à la surface solide avec une densité de population suffisante pour permettre aux molécules de l'agent biocompatible de protéger efficacement la surface solide par la surface biocompatible ;

   dans lequel le fragment de liaison chimique présente la formule A-X-B dans laquelle :
   - A représente un groupe photochimiquement réactif capable, en réponse à une activation spécifique, de se lier de façon covalente à la surface solide ;
   - B représente un groupe réactif différent, capable, en réponse à une activation spécifique à laquelle le groupe A est non sensible, de former une liaison covalente avec l'agent biocompatible ; et
   - X représente un fragment de squelette non interférant relativement inerte, réunissant les groupes "A", et "B", le fragment de squelette étant résistant au clivage dans des fluides physiologiques aqueux.

**2.** Procédé selon la revendication 1, dans lequel ledit groupe réactif différent est un groupe thermochimiquement réactif.

**3.** Procédé selon la revendication 1, dans lequel ledit groupe réactif différent est un groupe photochimiquement réactif.

**4.** Procédé selon la revendication 1, 2 ou 3, dans lequel le stimulus pour activer ledit groupe photochimiquement réactif est appliqué après l'application du stimulus pour activer ledit groupe réactif différent.

**5.** Procédé selon l'une des revendications précédentes, dans lequel la surface solide est essentiellement non réactive thermochimiquement.

**6.** Procédé selon la revendication 1, comprenant les étapes consistant à :
   - prendre un fragment de liaison chimique comprenant un groupe photochimiquement réactif capable, lors d'une activation, de se lier de façon covalente à ladite surface solide, et un groupe réactif différent, capable de se lier aux molécules de l'agent biocompatible, l'un des groupes étant non sensible à un stimulus auquel l'autre groupe réagit ;
   - activer ledit groupe réactif différent du fragment de liaison chimique en présence de molécules de l'agent biocompatible pour amener ledit groupe réactif différent à lier de façon covalente le fragment de liaison chimique auxdites molécules ; et
   - activer le groupe photochimiquement réactif du fragment de liaison chimique en présence de la surface solide du biomatériau pour amener le groupe photochimiquement réactif à se lier de façon covalente à la surface solide, avec une densité de population suffisante pour donner une surface biocompatible efficace.

**7.** Procédé selon la revendication 1, dans lequel X représente un groupe alkyle en $C_1$-$C_{10}$.

8. Procédé selon la revendication 1, dans lequel le groupe A est choisi parmi : les azides d'aryle, alkyle et acyle ; les dérivés alkyle et 2-cétodiazo et les diazirines (générateurs de carbène) ; les cétones aromatiques (générateurs d'oxygène triplet) ; les dérivés aromatiques de diazonium et les générateurs d'ions et radicaux carbonium.

9. Procédé selon la revendication 8, dans lequel le groupe A est choisi parmi : les azidonitrophényles, les fluoroazido nitrobenzènes et les cétones aromatiques.

10. Procédé selon la revendication 1, dans lequel le groupe B est un groupe thermochimiquement réactif choisi parmi : les halogénures de nitrophényle, les groupes alkylamino, alkylcarboxyle, alkylthiol, alkylaldéhyde, alkylméthylimidate, alkylisocyanate, alkylisothiocyanate et halogénure d'alkyle.

11. Procédé selon la revendication 1, dans lequel le groupe B est choisi parmi : les groupes carboxyle, les groupes hydroxyle, les groupes amino primaire, les groupes thiol, les maléimides et les groupes halogénure.

12. Dispositif biocompatible qui comprend :
    - un substrat d'un biomatériau ayant une surface solide et portant des molécules d'un agent biocompatible ;
    - un reste de fragment de liaison chimique, liant les molécules individuelles de l'agent biocompatible à la surface solide ;
    caractérisé par le fait que le reste du fragment de liaison chimique comprend un reste d'un groupe photochimiquement réactif lié de façon covalente à la surface solide, et comprend un reste d'un groupe réactif différent, lié de façon covalente aux molécules de l'agent biocompatible, le reste du groupe photochimiquement réactif étant lié à la surface solide de telle sorte que les molécules individuelles de l'agent biocompatible soient positionnées de façon suffisamment proche les unes des autres pour amener lesdites molécules à protéger de façon efficace la surface solide et à donner une surface biocompatible efficace,
    dans lequel le fragment de liaison chimique présente la formule A-X-B dans laquelle :
    - A représente un groupe photochimiquement réactif capable, en réponse à une activation spécifique, de se lier de façon covalente, à la surface solide ;
    - B représente un groupe réactif différent capable, en réponse à une activation spécifique, à laquelle le groupe A est non sensible, de former une liaison covalente avec l'agent biocompatible ; et
    - X représente un fragment de squelette, non interférant, relativement inerte, réunissant les groupes "A" et "B", le fragment de squelette étant résistante au clivage dans des fluides physiologiques aqueux.

13. Dispositif biocompatible selon la revendication 12, dans lequel ledit dispositif est approprié pour une implantation dans un corps.

14. Dispositif selon la revendication 12, dans lequel la surface solide est essentiellement non réactive thermochimiquement.

15. Dispositif selon la revendication 12, dans lequel l'agent biocompatible est un facteur de fixation cellulaire.

16. Dispositif selon la revendication 15, dans lequel le facteur de fixation cellulaire est la laminine ou la fibronectine.

17. Dispositif selon la revendication 12, dans lequel l'agent biocompatible est un facteur de croissance.

18. Dispositif selon la revendication 17, dans lequel le facteur de croissance est un facteur de croissance de l'endothélium, un facteur de croissance de l'épithélium, un facteur de croissance des ostéoblastes, un facteur de croissance des fibroblastes, un facteur de croissance issu des plaquettes, un facteur de croissance neural, ou un facteur de croissance du système circulatoire de l'embryon.

19. Dispositif selon la revendication 12, dans lequel l'agent biocompatible est le collagène.

**20.** Dispositif selon la revendication 12, dans lequel l'agent biocompatible est l'albumine.

**21.** Dispositif selon la revendication 12, dans lequel l'agent biocompatible est un polymère hydrophile.

**22.** Dispositif selon la revendication 21, dans lequel le polymère hydrophile est le polyéthylène glycol, l'acide hyaluronique, la méthyl cellulose, le collagène, le sulfate de chondroïtine ou le chitosan.

**23.** Dispositif selon l'une des revendications 12 à 22, dans lequel la surface solide est un tube pour greffe vasculaire, un tube ou une membrane de dialyse, un tube ou une membrane d'oxygénation du sang, une membrane d'ultrafiltration, une poche de sang, un cathéter, un ballon intra-aortique, une suture, une prothèse de tissu souple ou dur, une prothèse de synthèse, un organe artificiel ou une lentille pour l'oeil.

**24.** Dispositif selon la revendication 12, dans lequel lesdites molécules de l'agent biocompatible sont liées entre elles pour former un film biocompatible.

**25.** Dispositif selon la revendication 12, dans lequel l'agent biocompatible est un agent antimicrobien.

**26.** Dispositif selon la revendication 25, dans lequel l'agent biocompatible est le lysozyme ou la pénicilline.

**27.** Dispositif selon la revendication 12, dans lequel l'agent biocompatible est un agent antithrombogène.

**28.** Dispositif selon la revendication 27, dans lequel l'agent antithrombogène est l'héparine, la streptokinase, un activateur plasminogène tissulaire ou l'urokinase.

**29.** Dispositif selon la revendication 12, dans lequel le groupe X de l'agent biocompatible est un polyéthylène glycol ou un polypeptide.

**30.** Dispositif selon la revendication 12, dans lequel l'agent biocompatible est un acide gras.

**31.** Dispositif selon l'une des revendications 12 à 30, dans lequel la surface solide est un substrat polymère.

**32.** Dispositif selon la revendication 31, dans lequel le substrat polymère est un polyuréthanne, une polyvinylpyrrolidone, un polyamide, une polyamine, un polytétrafluoroéthylène, un poly(p-phénylènetéréphtalamide), un poly(chlorure de vinyle), un polypropylène, un polyacrylate, un polyméthacrylate, un polyacrylamide, une polyimine, un polyester, un polyéthylène, une cellulose ou une silicone.

**33.** Dispositif selon la revendication 23, dans lequel la lentille pour l'oeil est une lentille de contact ou une lentille intra-oculaire.

**34.** Dispositif biocompatible selon la revendication 12, dans lequel le dispositif est un dispositif de culture cellulaire ayant une surface solide portant des molécules d'un agent biocompatible choisi dans le groupe constitué par les facteurs de croissance, les facteurs de fixation cellulaire, les polymères de synthèse, les agents antithrombogènes, les glucides, les acides gras, et les agents antimicrobiens, et un reste de fragment de liaison chimique liant de façon covalente les molécules individuelles de l'agent biocompatible à la surface solide, le reste du fragment de liaison chimique incluant un reste d'un groupe photochimiquement réactif lié de façon covalente à la surface solide, et un reste d'un groupe réactif différent lié de façon covalente aux molécules de l'agent biocompatible, l'un des groupes étant non sensible à un stimulus auquel l'autre groupe réagit, le reste du groupe photochimiquement réactif étant lié à la surface solide de telle sorte que les molécules individuelles de l'agent biocompatible soient positionnées de façon suffisamment proche les unes des autres pour amener lesdites molécules à protéger de façon efficace la surface solide et à donner une surface biocompatible efficace.

**35.** Dispositif de culture cellulaire selon la revendication 34, dans lequel le dispositif est présenté sous une forme choisie dans le groupe constitué par des plaques, des feuilles et des tubes.

**36.** Dispositif de culture cellulaire selon la revendication 34, dans lequel le dispositif fournit une surface polymère pour la fixation cellulaire, choisie dans le groupe constitué par le poly(chlorure de vinyle), le polyéthylène, le polypropylène, le polyfluorotétraéthylène, les élastomères de silicone, le polyuréthanne, le polystyrène, et les polyacrylates y compris les polyméthacrylates.

**37.** Dispositif de culture cellulaire selon la revendication 34, dans lequel l'agent biocompatible est un facteur de croissance choisi dans le groupe constitué par la fibronectine, la laminine, le collagène, le facteur de croissance de l'endothélium, et l'albumine de sérum humain.

**38.** Dispositif de culture cellulaire selon la revendication 34, dans lequel le polymère de synthèse est un polymère de synthèse hydrophile.

**39.** Procédé selon la revendication 1, dans lequel X représente un groupe alkyle en $C_{1-10}$ tel que le polyméthylène ; un glucide tel que le polyméthylol ; un polyoxyéthylène tel que le polyéthylène glycol ; ou un polypeptide tel que la polylysine.

**40.** Dispositif selon la revendication 12, dans lequel A est choisi parmi : les azides d'aryle, alkyle, acyle; les dérivés alkyle et 2-cétodiazo et les diazirines (générateurs de carbène) ; les cétones aromatiques (générateurs d'oxygène triplet) ; les dérivés aromatiques et les générateurs d'ions et radicaux carbonium.

**41.** Dispositif selon la revendication 40, dans lequel le groupe A est choisi parmi : les azidonitrophényles, les fluoroazidonitrobenzènes et les cétones aromatiques.

**42.** Dispositif selon la revendication 12, dans lequel le groupe B est un groupe thermochimiquement réactif choisi parmi : les halogénures de nitrophényle, les groupes alkylamino, alkylcarboxyle, alkylthio, alkylaldéhyde, alkylméthylimidate, alkylisocyanate, alkylisothiocyanate et halogénure d'alkyle.

**43.** Dispositif selon la revendication 12, dans lequel le groupe B est choisi parmi : les groupes carboxyle, les groupes hydroxyle, les groupes amino primaire, les groupes thiol, les maléimides, et les groupes halogénure.

**44.** Dispositif selon la revendication 12, dans lequel X représente un groupe alkyle en $C_1$-$C_{10}$.

**45.** Dispositif selon la revendication 12, dans lequel X représente : un groupe alkyle en $C_{1-10}$ tel que le polyméthylène ; un glucide tel que le polyméthylol ; un polyoxyéthylène tel que le polyéthylène glycol ou un polypeptide tel que la polylysine.

**46.** Dispositif selon la revendication 29, dans lequel le polypeptide est la polylysine.